# EUROPEAN PATENT APPLICATION

(11) **EP 4 342 478 A1**
(43) Date of publication of application: **27.03.2024**
(21) Application number: 22804760.1
(22) Date of filing: 19.05.2022
(51) Int. Cl.: A61K 35/28, A61K 9/06, A61K 9/08, A61K 9/107, A61K 9/19, A61K 35/32, A61K 38/19, A61P 17/00

(54) **PREVENTIVE AGENT AND/OR THERAPEUTIC AGENT FOR BEDSORES**

(30) Priority: 19.05.2021 JP 2021084260
(71) Applicant: Tokyo Medical University, Tokyo 160-8402 (JP); Cysay Inc., Tokyo 105-0001 (JP)
(72) Inventor: YOSHIMOTO Takayuki, Tokyo 160-8402 (JP); KATAHIRA Yasuhiro, Tokyo 160-8402 (JP); YAMASHITA Yasuhiro, Tokyo 105-0001 (JP)
(74) Representative: Kuhnen & Wacker Patent- und Rechtsanwaltsbüro PartG mbB
(86) International application number: PCT/JP2022/020880
(87) International publication number: WO 2022/244851

(57) **Abstract**

The object of the present invention is to provide a preventive and/or therapeutic agent for pressure ulcers comprising a conditioned medium of immortalized mesenchymal stem cells to which specific genes are introduced as an active ingredient.

The present invention provides the preventive and/or therapeutic agent for pressure ulcers comprising the conditioned medium of immortalized mesenchymal stem cells, to which hTERT or pTERT gene, bmi-1 gene, HPV-E6 gene, and HPV-E7 gene are introduced, as an active ingredient. Here, the mesenchymal stem cells are preferably obtained from either human or swine. The conditioned medium preferably contains at least three or more cytokines selected from the group consisting of insulin-like growth factor binding protein, metalloproteinase tissue inhibitor, vascular endothelial growth factor, hepatocyte growth factor, tumor necrosis factor receptor II, and osteoprotegerin/osteoclast differentiation inhibitory factor; and each in the concentration range thereof is from 1 x 10³ to 1 x 10⁶ pg/mL, respectively.

## Description

### Technical field

The present invention relates to a preventive and/or therapeutic agent for pressure ulcer comprising a conditioned medium of mesenchymal stem cells as an active ingredient. In more detail, the present invention relates to the preventive and/or a therapeutic agent for pressure ulcer comprising the conditioned medium of immortalized mesenchymal stem cells prepared by introducing genes.

### Background Art

Elder people having a variety of diseases tend to increase in the current society due to aging. There is a problem of pressure ulcer, when the elder people are bedridden. The term, "pressure ulcer", is referred to as reddish part of skin, sore or wound formed by hindrance or obstruction of blood flow due to pressure of body weight caused by being bedridden. In general, it is referred to as "bedsore".

In the case of healthy people, they unconsciously perform "positional changes" such as turning over even during sleep or lifting their buttocks when sitting in a chair for a long time so that pressure is not applied to the same part of the body for a long time.

However, if they cannot perform positional changes by themselves, oxygen supply or nutrition supply becomes partially insufficient to the sites on which the pressure from the body weight is given to cause the formation of a "pressure ulcer". Sometimes the "pressure ulcer" is formed not only on the skin surface, but also inside the skin caused by damaged tissue located nearby the bone.

Not only bedridden patients for a long time but also patients having a weakened immune system due to side effects of drugs such as anticancer agents or steroids, or having disease such as congestive heart failure, pelvic fracture, spinal cord injury, diabetes, cerebrovascular disease or chronic obstructive pulmonary disease are susceptible to have pressure ulcer so that to be paid attention. This is because formed pressure ulcer functions as an infection route for microorganisms. Therefore, depending on the type of pressure ulcer that has formed, conservative treatment such as the application of agents, covering with dressings and the like, or surgical treatment such as excision thereof and tissue reconstruction is given to the patients.

Macrogol ointments such as Zinc oxide ointment, Orsenone (registered trademark) ointment, Riflap (registered trademark) ointment, Actosin (registered trademark) ointment, or compounds such as tretinoin tocopheryl, lysozyme hydrochloride, and the like, are used as therapeutic agents for pressure ulcer combined with other agents depending on the condition of the site on which pressure ulcer is formed.

On the other hand, therapeutic methods using biomedicines utilizing biological materials are recently employed. In 2015, human allogeneic bone marrow mesenchymal stem cells (herein below sometimes it is referred to as "MSCs".) is approved as the first regenerative medicine product in Japan for the treatment of acute graft-versus-host disease (herein below it is referred to as "prior art 1") according to the following reasons: MSCs are somatic stem cells belonging to the mesenchymal lineage and are collected from bone marrow, adipose tissue, umbilical cord, cord blood, dental pulp, and the like; they have the potency to differentiate into mesenchymal cells such as osteoblasts, adipocytes, muscle cells, and cartilage cells and the like; and also have the properties of immunosuppression or easy accumulation in inflammatory sites in damaged tissue; and show the potency of tissue repairment and regeneration. By this, MSCs are attracting attention as cell therapy for regenerative medicine.

According to the Ministry of Health, Labour and Welfare, "regenerative medicine" in the broad definition of the term refers to medical treatment that restores lost functions by activating the self-regenerative capacity of damaged organs through transplantation of devices incorporating living cells or activation/differentiation of intrinsic stem cells with cell growth and differentiation factors in the patient's body. More specifically, it is defined as the medicine to recover the lost physical function by growing stem cells which are externally cultured outside of the patient's body being transplanted into the patient's body to regenerate the damaged organs or tissues; and medicine to recover the lost physical function by growing stem cells which are externally cultured outside of the patient's body for artificially manufacturing the tissues, which are being transplanted into the patient's body to regenerate the damaged organs or tissues.

At present, the basic fibroblast growth factor (herein below, it is sometimes referred to as "bFGF") is put on the market as agents for pressure ulcer (refer to non-patent document #1). Also, it has been reported that MSCs, derived from bone marrow or umbilical cord, show the effectiveness on pressure ulcer model, if they are used for cell therapy. Moreover, it has been reported that MSCs by themselves relate to the treatment of pressure ulcer through the inhibitory effect of endoplasmic reticulum stress (refer to non-patent document #2, herein below, it is referred to as "prior art 2"). Here, endoplasmic reticulum stress is defined as a phenomenon caused by an accumulation of denatured proteins or misfolded proteins (herein below, they are referred to as "wrong proteins") due to improperly misfolded proteins in the endoplasmic reticulum (herein below it is abbreviated as "ER"). It has been reported that such wrong proteins relate to neurodegeneration diseases such as Parkinson's disease, Alzheimer's disease and the like, inflammation, cancer, and so on. Also, it is known that ER stress causes activation of some signal transduction pathways called ER stress response (UPR: unfolded protein response).

Also, an art for manufacturing cell lines transfected with immortalized genes (hTERT, HPV16 E6/E7, hBMI1) from dental pulp stem cells derived from human deciduous teeth as MSCs is known (refer to patent document #1, WO2017/078176. Herein below referred to as "prior art 3".).

### Prior art documents

### Patent documents

Patent document #1: WO 2017/078176

### Non-patent documents

Non-patent document #1: Robson M, et al. The safety and effect of topically applied recombinant basic fibroblast growth factor on the healing of chronic pressure sores Ann Surg.1992 216:401-406.
Non-patent document #2: Motegi S, et al., Protective effect of mesenchymal stem cells on the pressure ulcer formation by the regulation of oxidative and endoplasmic reticulum stress. Sci Rep. 2017 7(1):17186.
Non-patent document #3: Y. Yukie, et al. Thickness and pressure deformability of soft tissue over the sacrum in elderly and young subjects. Journal of International Nursing Research vol.27, No.2 2004

### Summary of invention

### Problems to be solved by the invention

Prior art 1 is superior in terms of using human allogenic MSCs for the treatment of acute graft-versus-host disease. However, the prior art 1 has problems that its application way is limited to the treatment of acute graft-versus-host disease, and it leaves the possibility to cause immune response due to the use of allogenic cells.

Prior art 2 is superior in terms of effectiveness to pressure ulcer models, when bone marrow- or umbilical cord-derived MSCs is used for cell therapy. However, the prior art 2 has problems that the MSCs used here are the cells by themselves so that it leaves the possibility of immune response similar to the prior art 1.

Also, when MSCs are used in cell therapy, some mechanisms are thought such as replacing damaged cells with the MSCs through their migration to inflammatory locations as well as paracrine effects of trophic factors such as cytokines, growth factors and the like secreted from the MSCs. However, there is the problem that the variety and production amounts of these trophic factors are different depending on the used cell populations.

Prior art 3 is superior in terms of the production of cell lines to which the immortalizing genes (hTERT, HPV16 E6/E7, hBMI1, and so on) are introduced. However, it is not expected in prior art 3 that the obtained immortalized stem cells or their conditioned medium are used to treat and/or prevent pressure ulcers.

Therefore, there are strong social needs for the agents to prevent and/or treat pressure ulcers with high efficiency and safety.

### Means for Solving the Problems

The present invention is accomplished under the above-mentioned situation and aims to provide preventive and/or therapeutic agents comprising a conditioned medium of immortalized MSCs introduced with specific genes as an active ingredient.

That is, the first embodiment of the present invention is a preventive and/or therapeutic agent for pressure ulcer comprising the conditioned medium of immortalized mesenchymal stem cells as an active ingredient, wherein bmi-1 gene, HPV-E6 gene, HPV-E7 gene, and TERT gene are introduced into the mesenchymal stem cells. It is preferable that the the said TERT is either hTERT or pTERT. Also, it is preferable that the mesenchymal stem cells are dental pulp stem cells. Furthermore, it is preferable that the dental pulp stem cells are obtained from human or swine.

Also, it is preferable that the conditioned medium comprises at least 3 cytokines selected from the group consisting of insulin-like growth factor-binding protein, tissue inhibitor of metalloproteinase, vascular endothelial growth factor, hepatocyte growth factor, tumor necrosis factor receptor II, and osteoprotegerin osteoclast differentiation inhibitory factor in a concentration range from 1 × 10³ to 1 × 10⁶ pg/mL.

Furthermore, the insulin-like growth factor-binding protein is at least one selected from the group consisting of IGFBP-2, IGFBP-3, IGFBP-4, and IGFBP-6. Also, it is preferable that the tissue inhibitor of metalloproteinase is TIMP-1 or TIMP-2.

It is preferable that the conditioned medium is any one of forms selected from the group consisting of frozen form, lyophilized powder form, and liquid form. Also, it is preferable that the preventive and/or therapeutic agent for pressure ulcer further comprises one or more components selected from the group consisting of excipient, pH regulator, diluent, and buffer as needed.

It is preferable that dosage form of the preventive and/or therapeutic agent is any one selected from the group consisting of injections, injections, ointment, plaster, cream, and transdermal form.

### Advantageous Effect of the Invention

According to the present invention, the preventive and/or therapeutic agent for pressure ulcer, it prevents pressure ulcer occurrence or accelerates quick recovery thereof after pressure ulcer is formed, because it comprises the conditioned medium of immortalized MSCs to which bmi-1, HPV-E6, HPV-E7, and TERT genes are introduced with as an active ingredient.

Also, according to the present invention, the preventive and/or therapeutic agent for pressure ulcer, the preventive and/or therapeutic agent has constant quality, because the use of the immortalized stem cells makes it possible to obtain the culture supernatant comprising constant types and amounts of components.

### Brief Description of the Drawings

[Fig. 1]

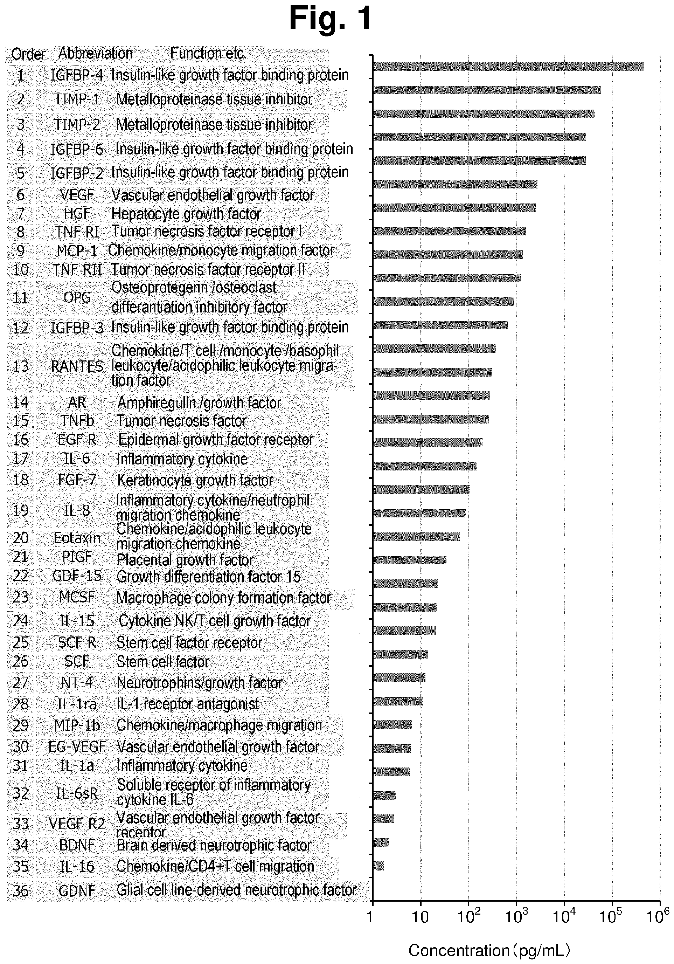

   Figure 1 is a graph showing the results of analysis for cytokines having not less than a certain concentration contained in the conditioned medium of immortalized MSCs (hSHED-CM) used in the present invention.
[Fig. 2]

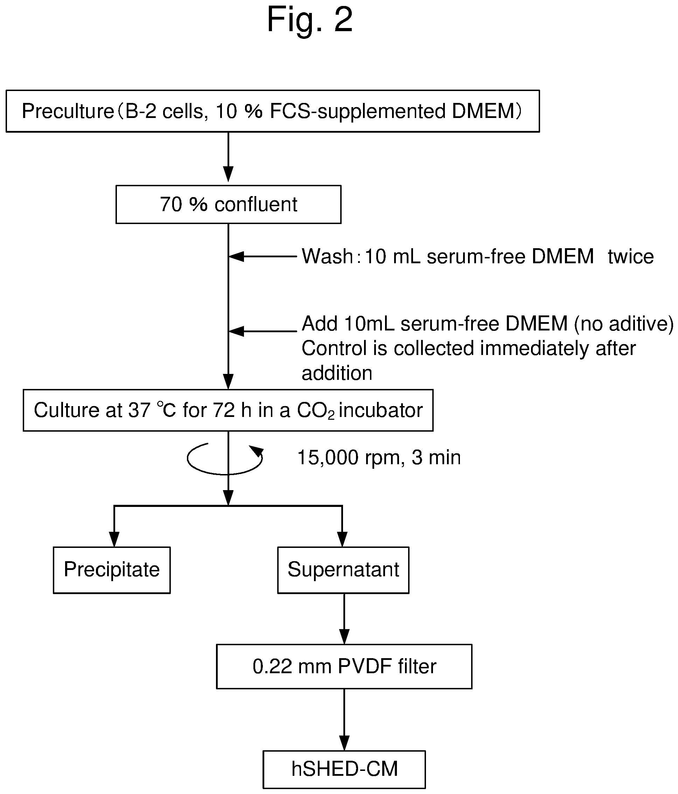

   Figure 2 is a flow chart showing the method for preparing the hSHED-CM as described above.
[Fig. 3]

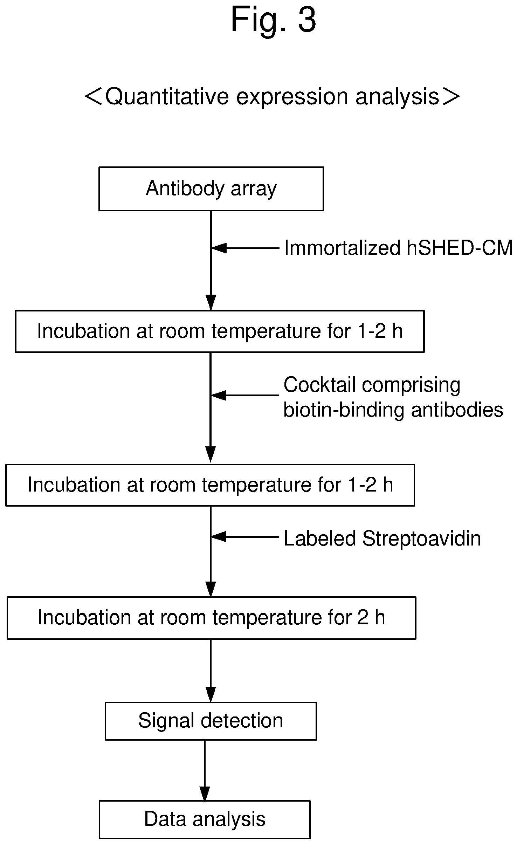

   Figure 3 is the flow chart showing the quantitative expression analysis of cytokines contained in the hSHED-CM.
[Fig. 4]

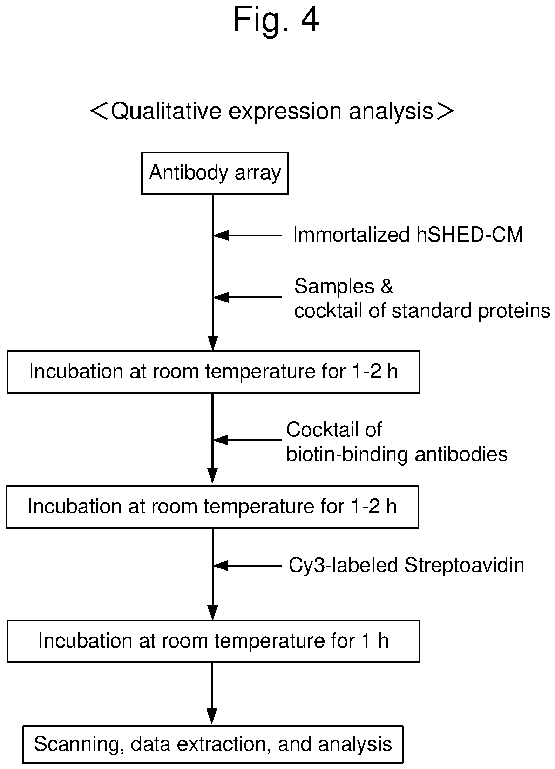

   Figure 4 is the flow chart showing the qualitative expression analysis of cytokines contained in the hSHED-CM.
[Fig. 5]

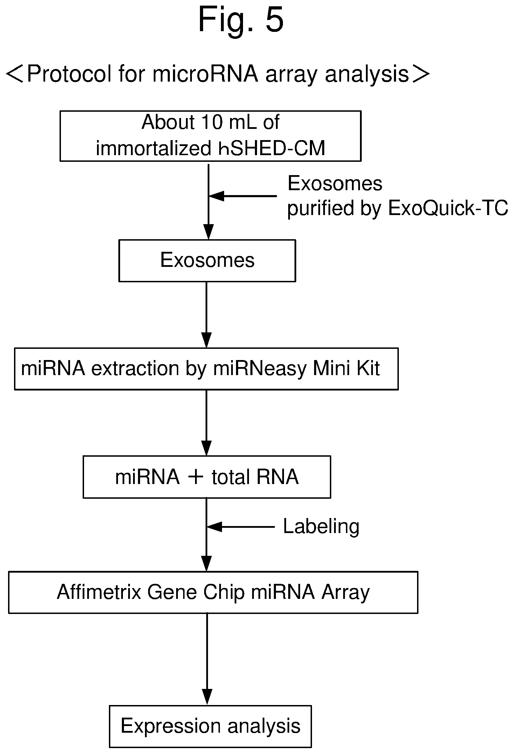

   Figure 5 is the flow chart showing the expression analysis of microRNA in exosomes acquired from the hSHED-CM (No. 1).
[Fig. 6]

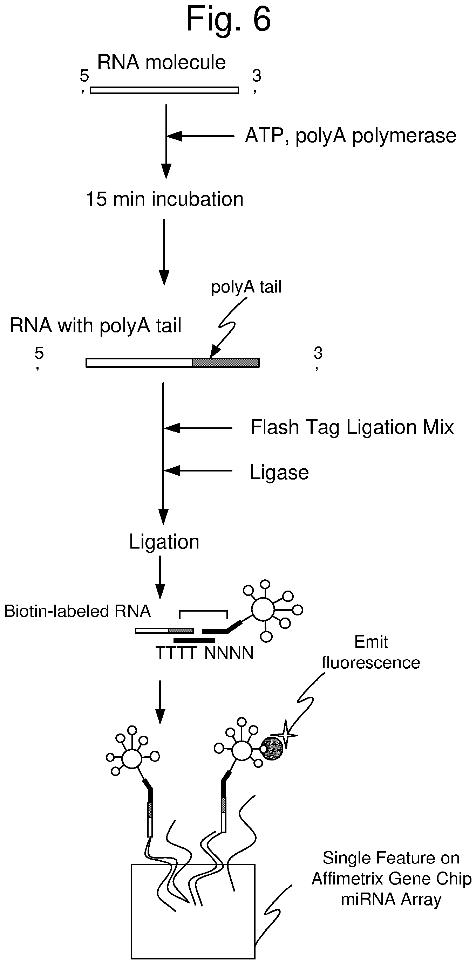

   Figure 6 is the flow chart showing the expression analysis of microRNA in exosomes acquired from the hSHED-CM (No. 2).
[Fig. 7]

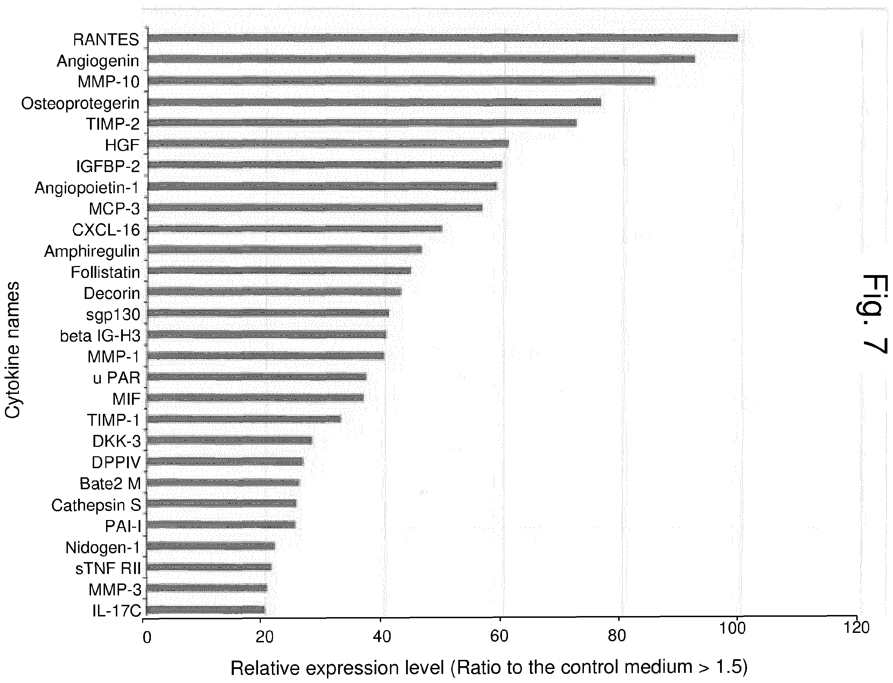

   Figure 7 is the graph showing the results of the qualitative expression analysis of cytokines acquired according to the processes in Figure 4 (No. 1).
[Fig. 8]

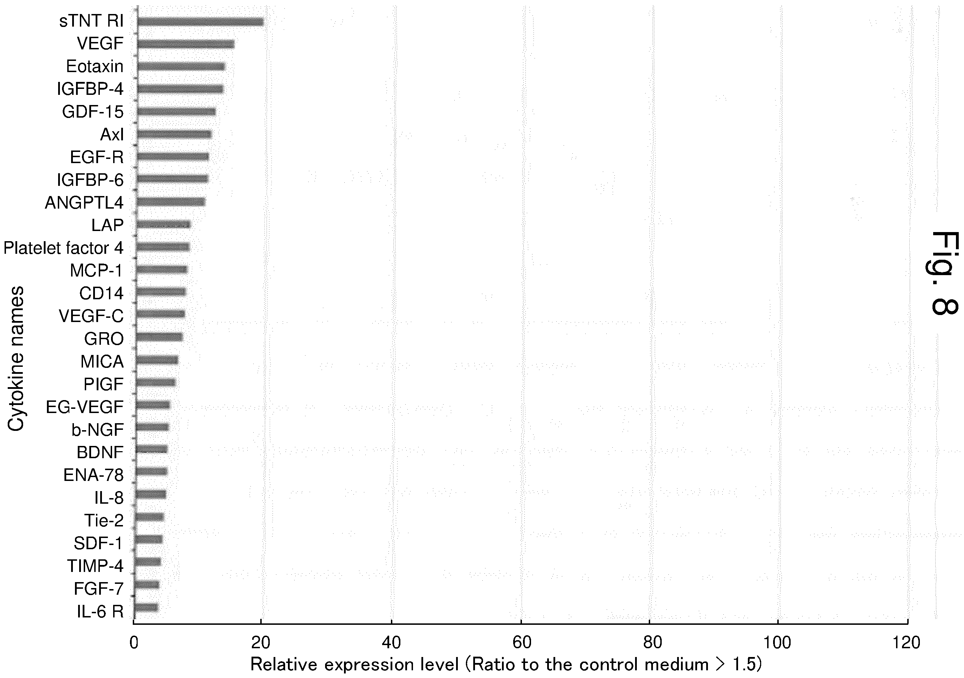

   Figure 8 is the graph showing the results of the qualitative expression analysis of cytokines acquired according to the processes in Figure 4 (No. 2).
[Fig. 9]

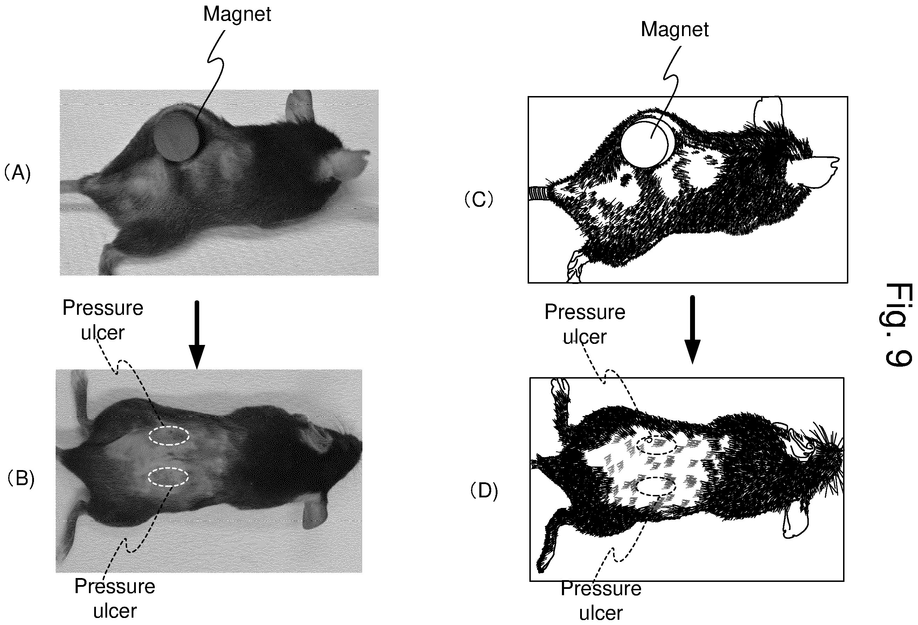

   Figure 9 shows (A) a protocol for experimental formation of pressure ulcer on a mouse, and (B) a photograph of developed pressure ulcer. Figures 9 (C) and (D) show traced drawings of the pressure ulcer photographs shown on Figure 9 (B).

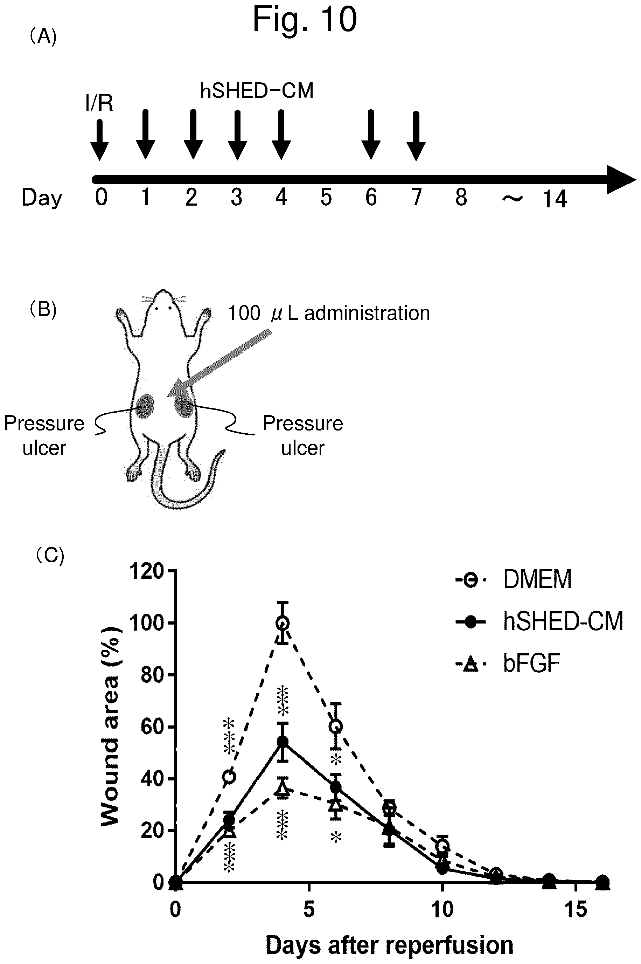

[Fig. 10] Figure 10 is a drawing showing the administration sites and administration results in the case of administration schedule 1. Figure 10 (A) shows the administration schedule 1 (4-day consecutive administration from the next day of the I/R treatment, one day blank, and then again 2-day consecutive administration), and Figure 10 (B) shows the administration sites. Figure 10 (C) is the graph showing the time-dependent change of the damaged area after reperfusion. In Figure 10 (C), DMEM represents Dulbecco-modified Eagle's medium, hSHED-CM represents the culture supernatant, conditioned medium, of the immortalized stem cells derived from human dental pulp stem cells, and bFGF represents the basic fibroblast growth factor respectively.
[Fig. 11]

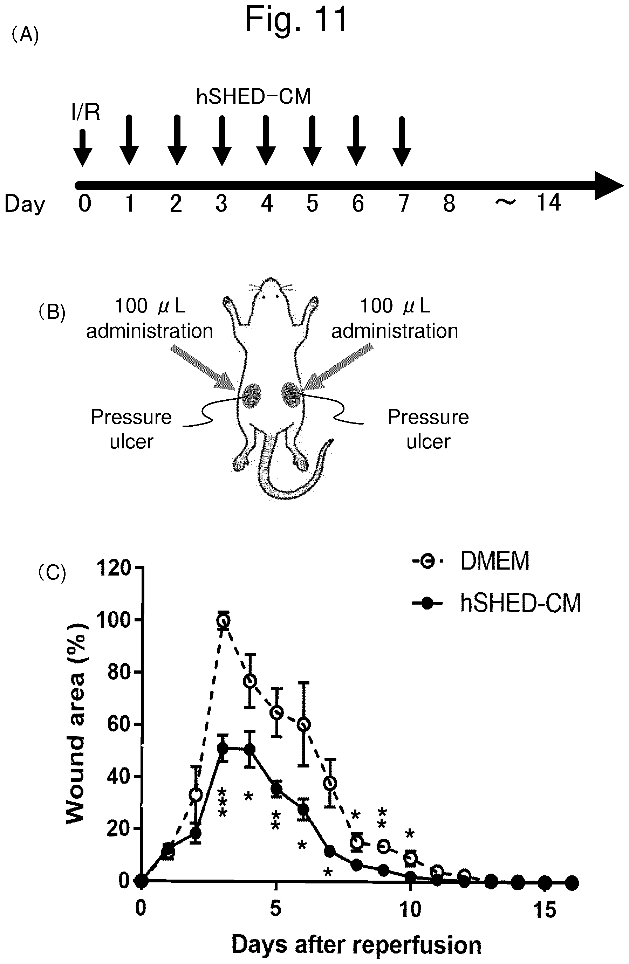

   Figure 11 is the drawing showing the administration sites and administration results in the case of administration schedule 2. Figure 11 (A) shows the administration schedule 2 (7-day consecutive administration from the next day of the I/R treatment) and Figure 11 (B) shows the administration sites. Figure 11 (C) is the graph showing the time-dependent change of the damaged area after reperfusion. In Figure 11 (C), DMEM represents Dulbecco-modified Eagle's medium, hSHED-CM represents the conditioned medium of the immortalized stem cells derived from the human dental pulp stem cells, respectively.
[Fig. 12]

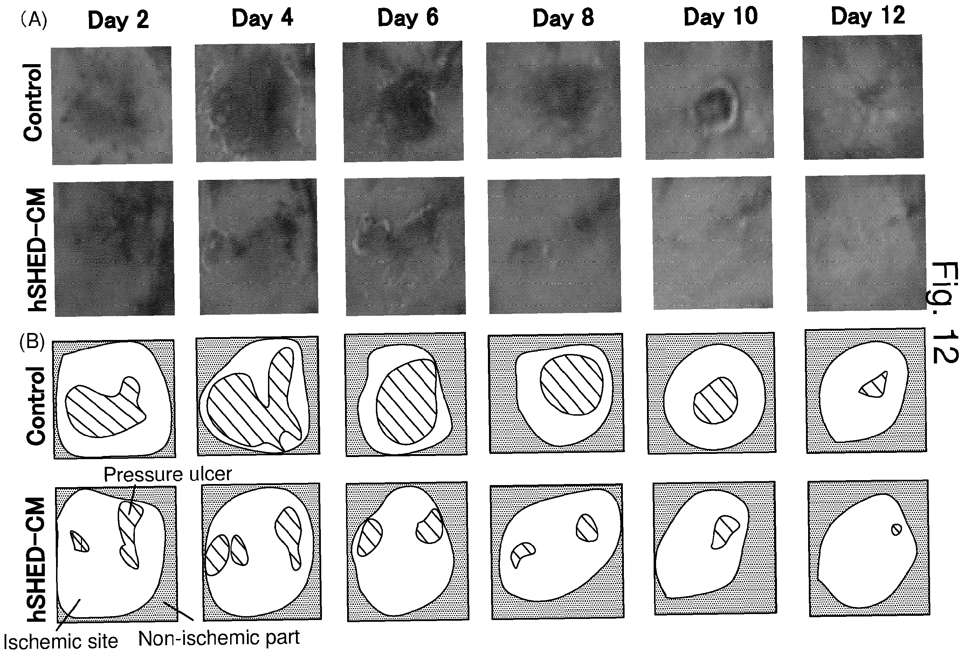

   Figure 12 (A) is the photograph showing the curing status of mouse pressure ulcer sites treated under the administration schedule shown in Figure 11 (A). Upper panels show changes in the I/R sites in the control mice from Day 2 to Day 12, and lower panels show the I/R sites in the hSHED-CM administration group in the same period. Figure 12 (B) shows schematic drawings of their images.
[Fig. 13]

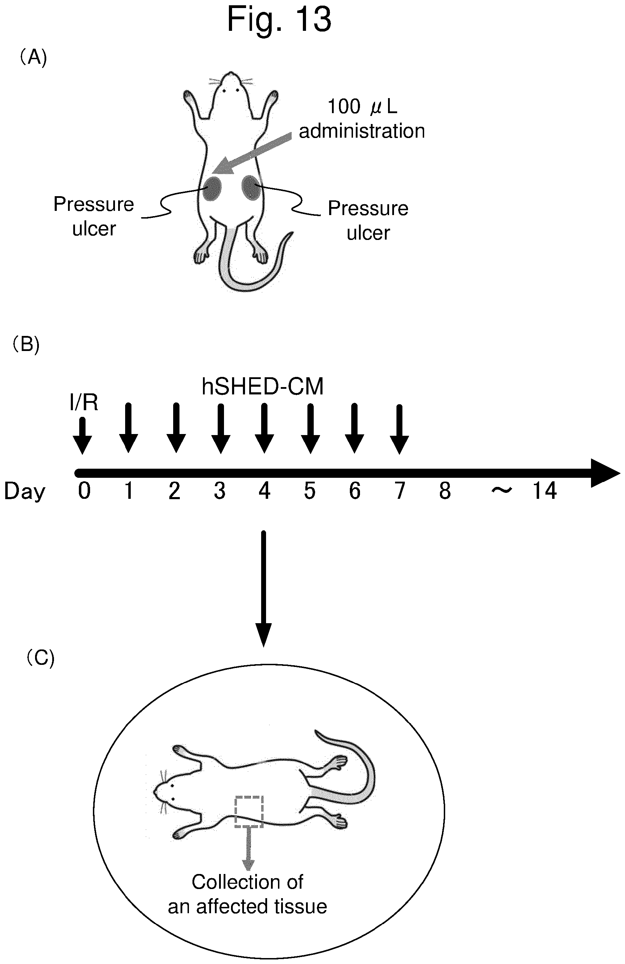

   Figure 13 is the drawing showing the administration sites and the administration results in the case of the administration schedule 3. Figure 13 (A) shows a part of the I/R treatment, wherein the red arrow indicates the injection of 100 µL to one of the I/R treatment parts. Figure 13 (B) shows the administration schedule 3 (7-day -consecutive administration from the next day after the I/R treatment). The affected tissue is collected from the part shown in Figure 11 (C) on day 4.
[Fig. 14]

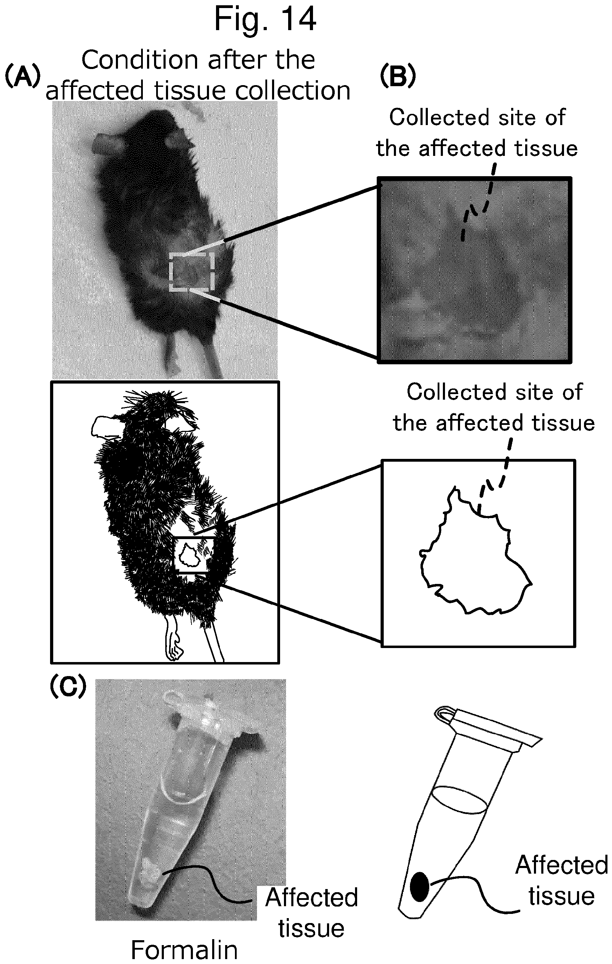

   Figure 14 shows the photographs: (A) the photograph showing the mouse from which the affected tissue shown in Figure 13 (C) was collected; (B) the magnified photograph of the tissue; and the photograph of the fixed tissue in formalin. Their line drawings are shown under the photographs ((A) and (B)) or right side (C).
[Fig. 15]

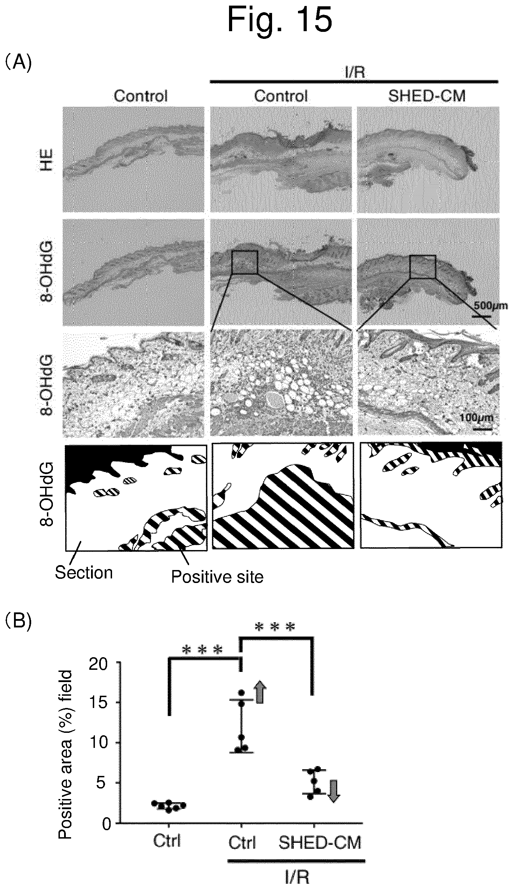

   Figure 15 shows the photographs: Fig. 15 (A), images of thin-sliced sections of the collected tissue (shown in Figure 14 (C)) which is embedded in paraffin and is stained with Hematoxylin Eosin (HE) or immunohistochemically stained for 8-OHdg, which is an indicator of the reactive oxygen. Fig. 15 (B) shows the graph of the quantified stained area. In Figure 15 (A), schematic drawings showing the magnified view of the staining images are shown in the fourth row from the top. In the drawings, blank parts indicate the section, hatched area indicates the positively stained area for 8-OHdG in the sections.
[Fig. 16]

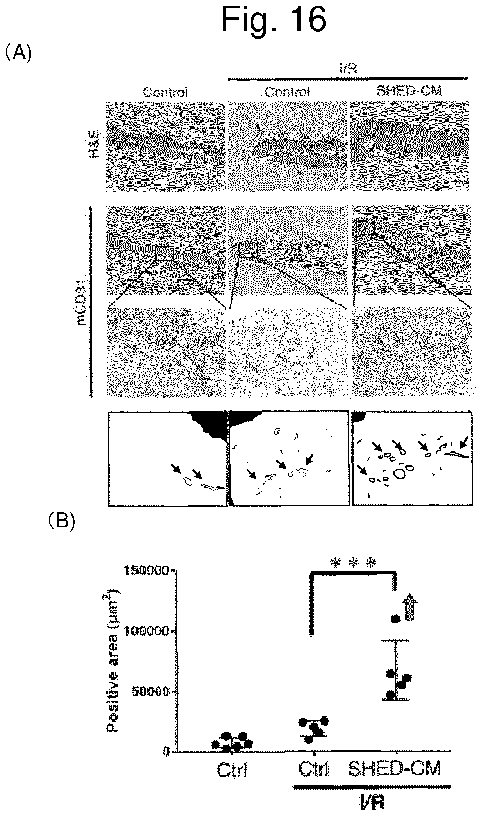

   Figure 16 shows the photographs: (A) the images of the thin-sliced sections of the collected tissue (shown in Figure 14) which is embedded in paraffin and is stained with HE or immunohistochemically stained with anti-CD31 which is an antibody against CD31, the marker of pericyte. Fig. 16 (B) is the comparison results of the quantified CD31 positive area. In Figure 16 (A), the schematic line drawings shown in the fourth row from the top show the magnified images in the third row. In the line drawings, blank parts indicate the section. The solid lines in the sections indicate the area positively stained with mCD31 positive area, which are emphasized by arrows.
[Fig. 17]

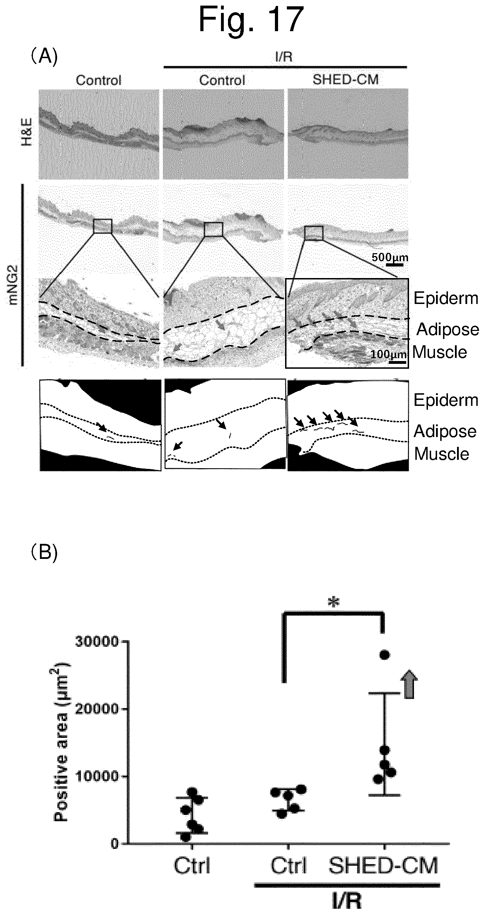

   Figure 17 shows the photograph (A), the images of the thin-sliced sections of the collected tissue (shown in Figure 14) which is embedded in paraffin, and is stained with HE or immunohistochemically stained with anti-NG2, which is the antibody against the marker of pericyte. Fig. 17 (B) shows the comparison results of the quantified NG2 positive area. In Figure 17 (A), the schematical drawings showing the magnified images in the third row from the top are shown in the fourth row from the top. In the line drawings, the blank area shows the section, wherein the black dashed lines indicate the border of tissues, solid short lines indicate the mNG2 positive parts, which are emphasized by arrows.
[Fig. 18]

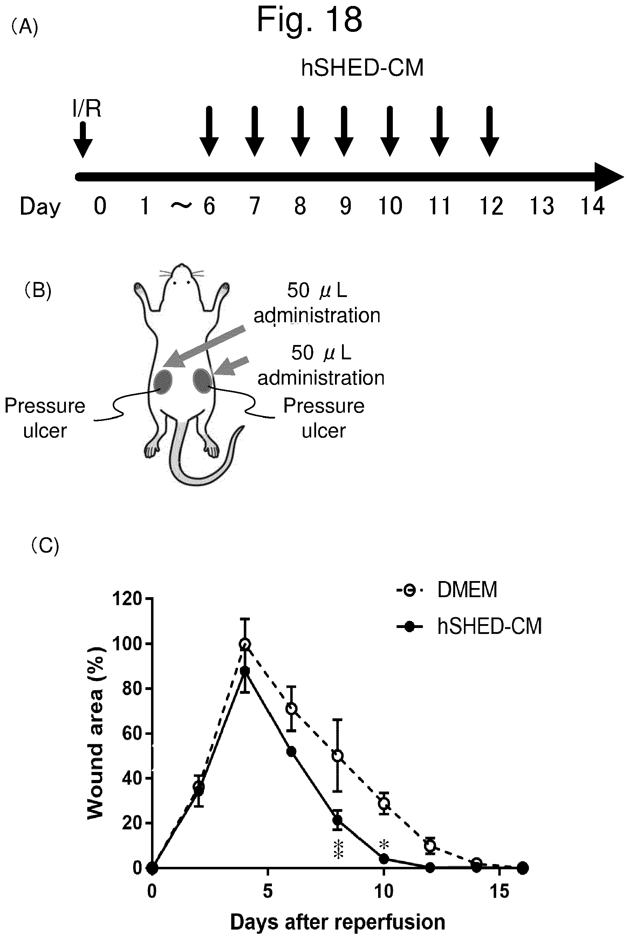

   Figure 18 is the drawings showing the administration sites and the administration results in the case of the schedule 4. Figure 18 (A) shows the administration schedule 4 (7-day consecutive administration after the pressure ulcer is developed) and Figure 18 (B) shows the administration sites. Figure 18 (C) shows the graph showing the time-dependent change of the damaged area. In Figure 18 (C), the arrows indicate the initial date of the administration. In the graph, the control medium indicates DMEM, and hSHED-CM indicates the conditioned medium of the immortalized stem cells derived from human dental pulp stem cells, respectively.
[Fig. 19]

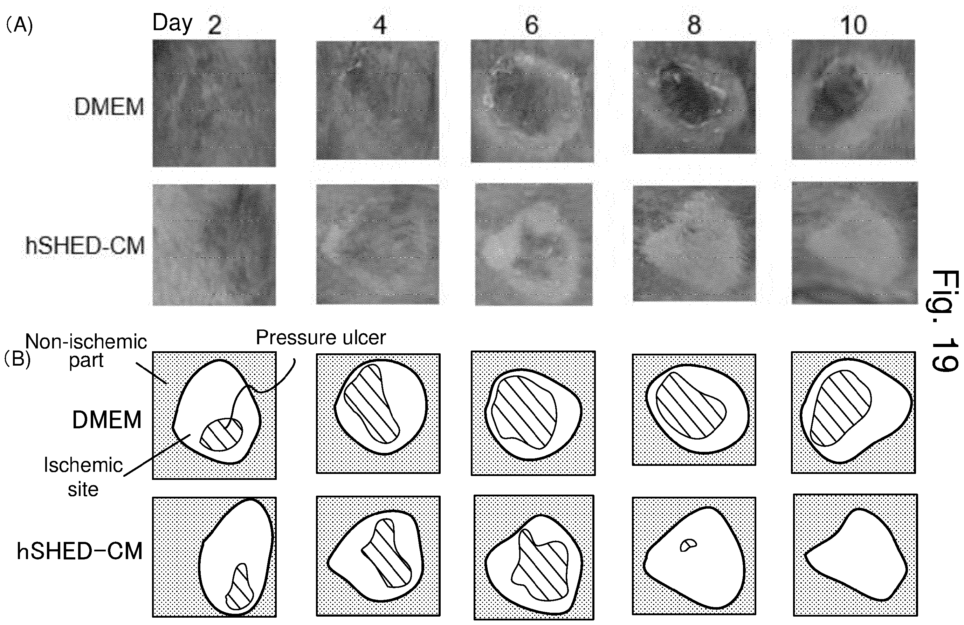

   Figure 19 (A) is the images showing the curing status of the mouse pressure ulcer sites which are treated according to the administration schedule shown in Figure 18 (A). The upper row is the images showing the time-dependent change of the pressure ulcer-formed sites (herein below, it is sometimes referred to as "I/R treatment") from the control group mice. The lower row is the images showing the time dependent changes of the sites from the hSHED-CM administrated group during the same time period. Figure 19 (B) is the line drawings schematically representing the images.
[Fig. 20]

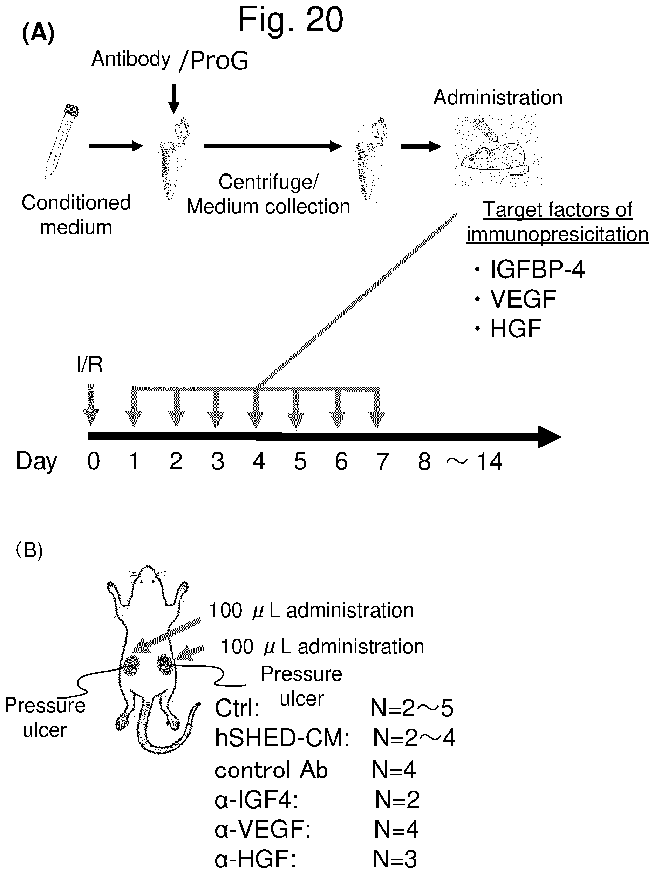

   Figure 20 is schematics showing the experimental design for administrating predetermined cytokines to the I/R treatment sites. Figure 20 (A) shows the administration schedule of the conditioned medium in which predetermined factors are depleted. Figure 20 (B) is a schematic showing the administration sites (the pressure ulcer sites) of the mouse.
[Fig. 21]

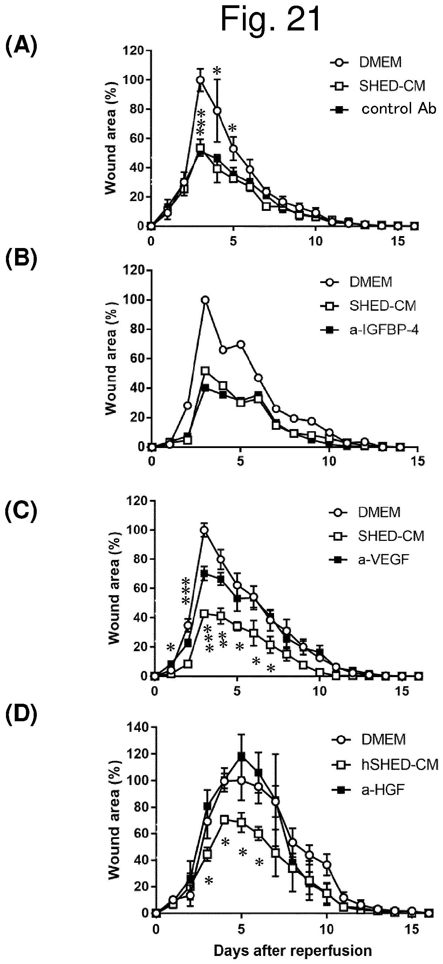

   Figure 21 includes graphs showing the time-dependent change of the pressure ulcer-formed area size when administrating each factor to the sites shown in Figure 20 (B) according to the administration schedule shown in Figure 20 (A). Figure 21(A) shows the result when the negative control (control antibody (control Ab)) was used, Figure 21(B) shows the result when the conditioned medium from which IGFBP-4 was depleted was used, Figure 21(C) shows the result when the conditioned medium from which VEGF was depleted was used, and Figure 21(D) shows the result when the conditioned medium from which HGF was depleted was used.
[Fig. 22]

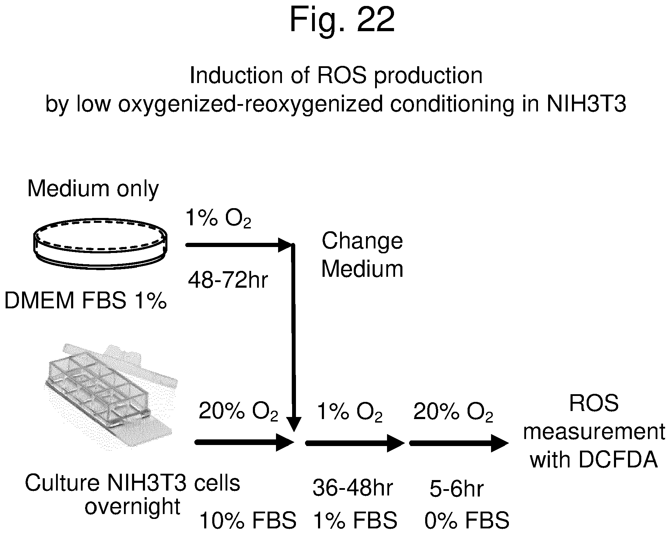

   Figure 22 is the schematic of the experimental process which reproduces the development mechanism of the pressure ulcer in vitro to show the inhibitory effect of the conditioned medium on reactive oxygen species (herein below, it is sometimes abbreviated as "ROS").
[Fig. 23]

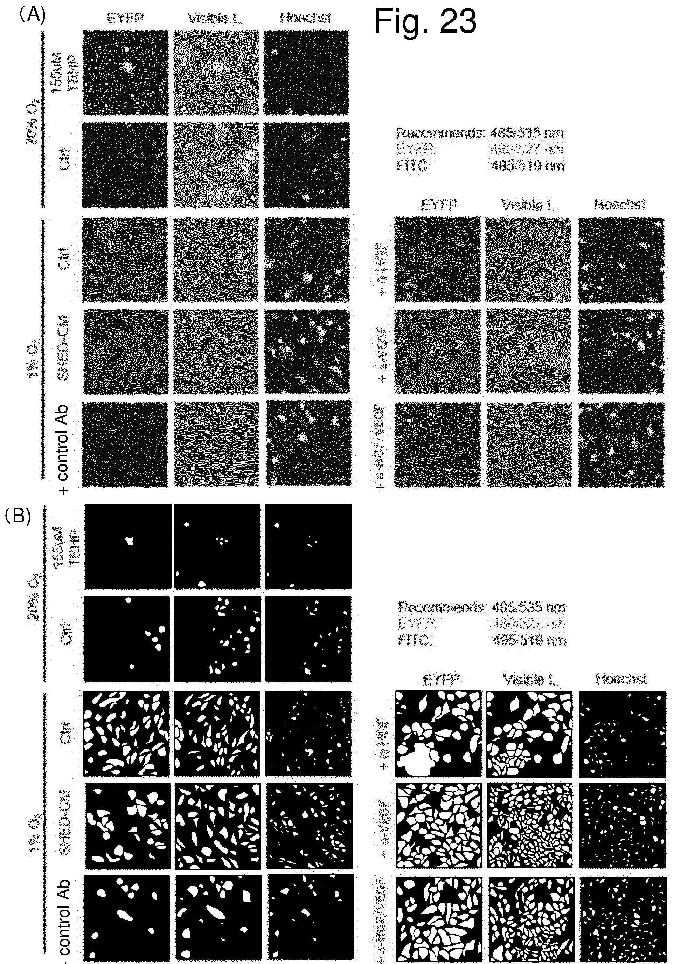

   Figure 23 shows the observation results of the fluorescence microscopy showing changes in ROS inhibition, when NIH3T3 was cultured with the conditioned medium from which HGF and/or VEGF were depleted by immunoprecipitation with specific antibodies in vitro experiment shown in Figure 22. Figure 23 (A) is their observation images and (B) is the line drawings thereof.
[Fig. 24]

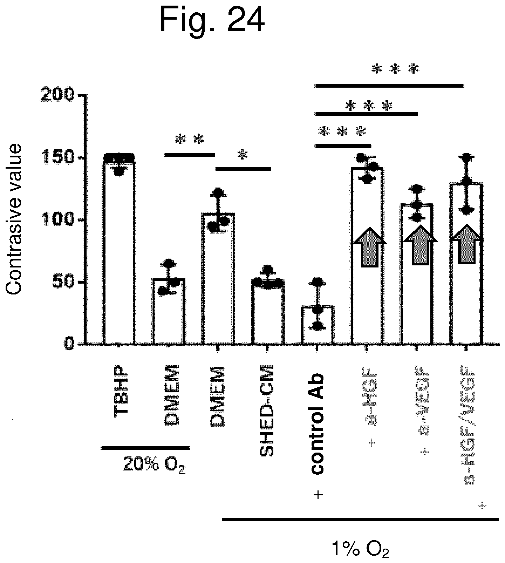

   Figure 24 is the graph that quantitatively compared the contrast as the relative value of ROS, wherein the contrast was calculated from the maximum and minimum brightness of the image shown in Figure 23.
[Fig. 25]

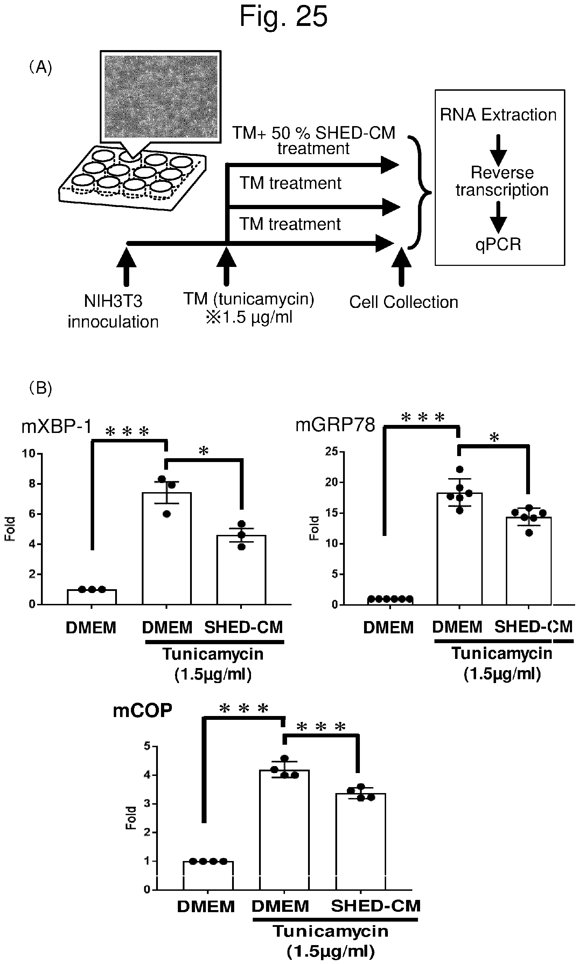

   Figure 25 shows the inhibitory effect on ER stress by the conditioned medium, in the case of mouse fibroblast strain, NIH3T3 cells. Figure 25 (A) is a schematic showing the processes to perform real-time PCR by adding 1.5 µg/mL of Tunicamycin (herein below, it is sometimes abbreviated as "TM"). Figure 25 (B) is the graph showing the expression level of the mRNAs of ER stress marker acquired by using real-time PCR with the collected and treated NIH3T3 cells.
[Fig. 26]

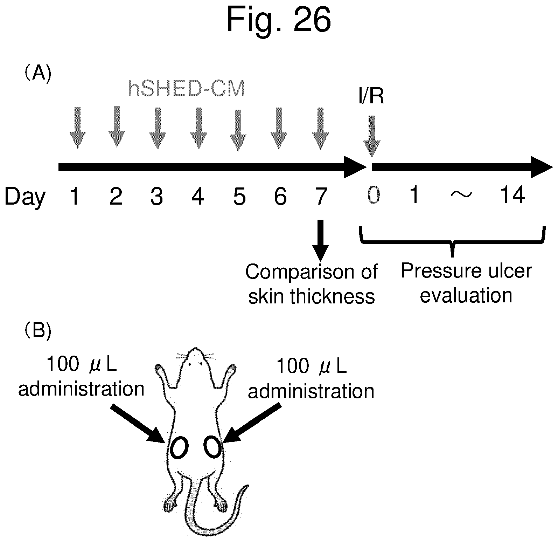

   Figure 26 is the schematic figure of the experiment for showing the inhibitory effect of the conditioned medium of the present invention against the pressure ulcer with prior dosing. Figure 26 (B) is the schematic figure showing the administration sites on the mouse to which SHED-CM is administered.
[Fig. 27]

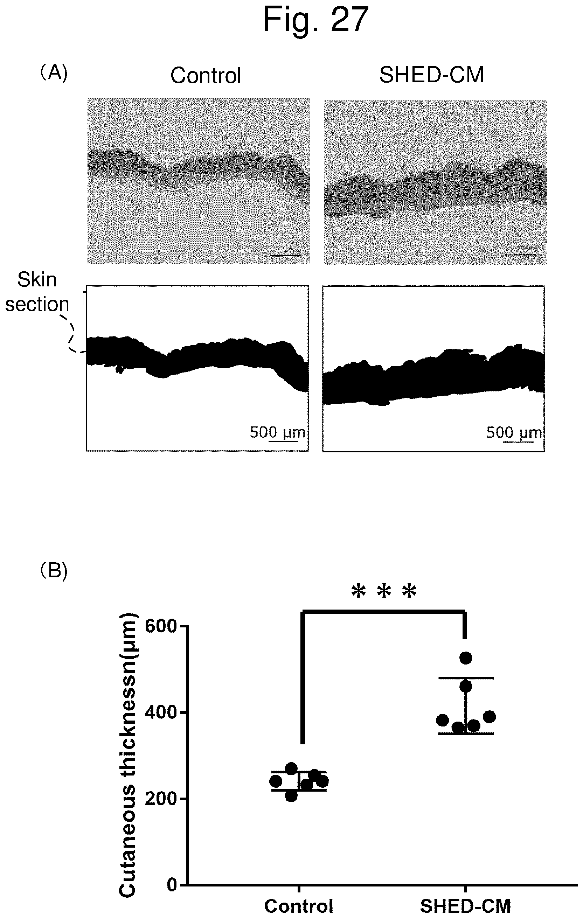

   Figure 27 shows the inhibitory effect of the pressure ulcer development by the prior dosing of the conditioned medium of the present invention. Figure 27 (A) is observation images and their line drawings showing the comparison results of the skin thickness after 7-day administration of the conditioned medium, the present invention. Figure 27 (B) is the graph showing the comparison results of the quantified thickness of the skin.
[Fig. 28]

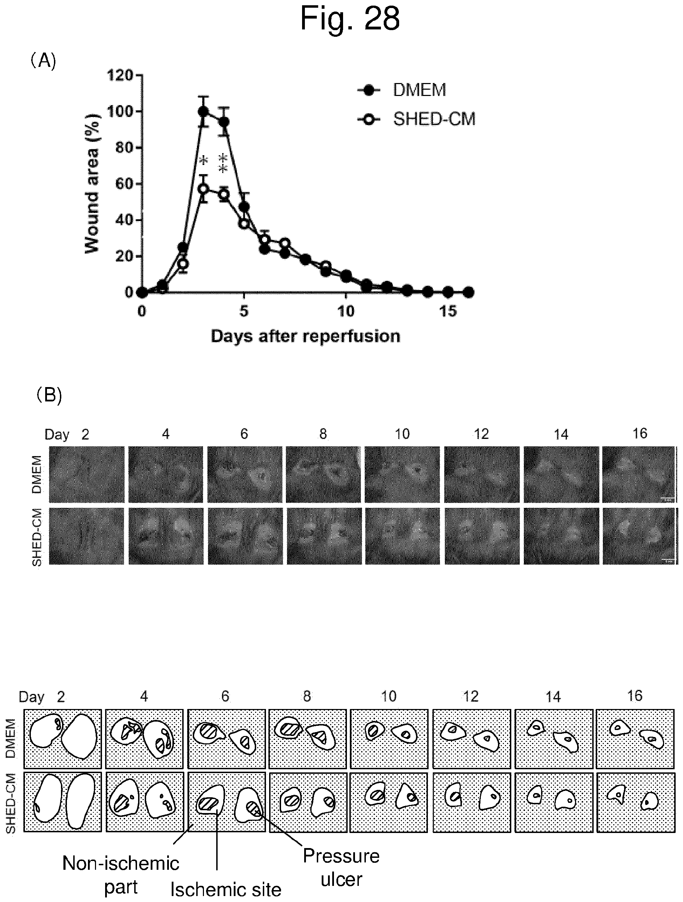

   Figure 28 is drawings showing the administration results when the administration schedule 6 shown in Figure 26 (A) was employed. Figure 28 (A) is the graph showing the time-dependent change of the damaged area. Figure 28 (B) is the images and schematic line drawings showing the curing status of the pressure ulcer formed on the mouse to which the conditioned medium was dosed in advance. The upper and lower rows show the photographs of the change of I/R treatment sites; the upper row is those of the control mouse group, and lower row is hSHED-CM administrated mouse group, respectively.

### Mode for carrying out the invention

Herein below, one aspect of the present invention is explained using Figures 1 to 28. In the following specifications and drawings, the same reference numerals are given to the same or equivalent elements to avoid redundant explanations.

The first aspect of the present invention, the preventive and/or therapeutic agent for the pressure ulcer is produced by the following processes. That is, (S1) producing immortalized stem cells by inducing a set of specific 4 genes to MSCs, (S2) preparing the conditioned medium by culturing the obtained immortalized MSCs, and (S3) producing the preventive and/or therapeutical agent for the pressure ulcer comprising the conditioned medium as an active ingredient.

First of all, in order to acquire the present invention, the immortalized stem cells, stem cells are isolated from mammal MSCs. It is preferable to select the mammal from the group consisting of human, swine, horse, and monkey, because genetic similarity is high compared with human cells and low risk of infection.

In this specification, the word, "MSCs", indicates cells having differentiation ability into cells belonging to the mesenchymal lineage, such as osteoblasts, adipocytes, myocytes, and chondrocytes. As specific mesenchymal cells, dental pulp cells, bone marrow cells, umbilical cord cells, and adipocytes from the above animals can be enumerated. Furthermore, "dental pulp cells" indicate one of the stem cells contained in the dental nerve having regeneration ability. They have the feature that their genes are less damaged because they are protected from ultraviolet light and radiation by hard material teeth.

The gene set described above is composed of bmi-1, HPV-E6, HPV-E7, and TERT. hTERT is a gene of telomere-repairing enzyme. bmi-1 is the gene of Bmi-1 protein that consists of a polycomb complex. Bmi-1 is necessary to maintain hematopoietic stem cells and acts to proliferate hematopoietic stem cells through activity enhancement. E6 and E7 are early genes of HPV-16 or HPV-18 DNA.

The following is a description for the preparation of the immortalized stem cells using dental pulp cells from human deciduous teeth.

First, the deciduous tooth is disinfected with, for example, chlorhexidine, isodine solution, or other disinfectants; and then the crown of the teeth is divided to collect the pulp tissue by using a dental reamer.

The harvested pulp tissue is then suspended in basal medium, for example, Dulbecco's Modified Eagle's Medium (hereafter referred to as "DMEM") containing 5-15 % calf serum (hereafter referred to as "CS") and 50 to 150 units/mL of antibiotics. Then, they are treated with 1 to 5 mg/mL collagenase and 1 to 5 mg/mL dispase at 37 °C for 0.5 to 2 hours.

In addition to DMEM, Iskov's modified Dulbecco's medium (IMDM) (GIBCO, etc.), Ham F12 medium (HamF12) (Sigma, GIBCO, etc.), RPMI 1640 medium, and the like may be used as the basal medium. Two or more basal media may be used in combination. As an example of the mixed medium, there is mentioned such as the mixture being composed of equal amounts of IMDM and HamF12 (e.g., commercially available as the trade name: IMDM/HamF12 (GIBCO)).

In addition, as additives to the basal medium, there are mentioned such as fetal calf serum (herein below referred to as "FCS"), human serum, sheep serum or other serum, serum substitutes (such as Knockout serum replacement (KSR)), bovine serum albumin (herein below referred to as "BSA"), penicillin, streptomycin, and other antibiotics, various vitamins, and various minerals.

The basal media may be also used for cell selection as described below, and for culturing cells after selection.

After the enzyme treatment, centrifugation (3,000 to 7,000 revolution/min) for 3 to 10 minutes is performed to collect the dental pulp cells. Depending on a necessity, the cells are selected by using the strainer. The selected cells are, for example, resuspended in 3 to 6 mL of the basal medium and then spread on an adherent cell culture dish having the diameter of 4 to 8 cm.

Subsequently, after adding a culture medium, for example, DMEM containing 10 % FCS to the dish, the cells are cultured in a 5 % CO₂ incubator at 37 °C for about 2 weeks. After removing the culture medium, the cells are washed out once to several times with such as PBS. Instead of both removing the medium and washing out the cells, adherent dental pulp stem cells forming colonies can be collected. The adherent dental pulp stem cells are, for example, detached from the dish by treating with 0.025 % to 0.1 % of trypsin and 0.3 to 1 mM EDTA at 37 °C for several minutes and then collected.

Next, the adherent cells selected as described above are cultured. For example, the dental pulp stem cells acquired as described above are spread on a culture dish for adherent cells and are cultured at 37 °C in a 5 % CO₂ incubator.

Passage is performed, for example, by detaching the cells from the culture dish with both of trypsin and EDTA, and collecting them as described above, when the cultured cells are proliferated subconfluently or confluently based on observation by necked eye. Then, they are spread on the culture dish containing the culture medium again.

Here, the term, sub-confluent, indicates the situation that cells attach to about 70 % surface of the culture dish. For example, the selected cells through the 1 to 8 passage are proliferated to necessary cell numbers, for example, 1 × 10⁷ cells/mL. After cultured as described above, the cells are collected and stored in liquid nitrogen. Dental pulp stem cells collected from several donors can be stored as a bank of dental pulp stem cells.

Next, 4 types of genes are introduced into the primary cultured cells acquired by primarily culturing the stem cells to produce gene-introduced cells. It is preferable that the introduced genes are hTERT, bmi-1, E6, and E7 to obtain the immortalized cells having greater doubling numbers. Here, hTERT is the gene for human telomerase reverse transcriptase, and bmi-1 is a polycomb group gene involved in the regulation of stem cell self replication and differentiation. E6 and E7 are genes used by human papillomaviruses for self replication in open reading frame coding early genes.

Such gene introduction may be conducted by the following procedures.

Plasmids to which the genes described above are inserted are prepared, and then they are incorporated into shuttle vectors, for example, pShuttle2, for cloning the genes. Then, the shuttle vector is used for transformation of E.coli for selecting kanamycin resistant transformant. The plasmid DNA in the selected kanamycin-resistant transformant E.coli are purified to be analyzed the restriction enzyme sites for identifying the recombinant one.

### 1. Steps for preparations of DNA fragments and virus vectors

### 1.1 Preparation of DNA fragments to be introduced into viruses

First, sequence information of vectors used for inserting DNA fragments into viruses is acquired. As viruses used for vectors for gene introduction in the present method, any one of the viruses is preferably selected from the group consisting of lentivirus, adenovirus, and retrovirus, because the introduced genes are not transiently but stably expressed. Lentivirus is preferable due to the high efficiency of the production rate of the strains stably expressing the introduced genes.

In the following, as the example, the steps are explained when lentivirus plasmid vector (pLVSIN) is used. The sequence information of pLVSIN is downloaded, for example, from the TAKARA bio web catalogue to confirm the multi-cloning sites.

Next, DNA fragments are prepared as follows. In the present invention, the DNA fragments to which 2 to 4 genes are inserted are used to prepare the immortalized stem cells. Cells used here are not limited unless they are stem cells from mammals. However, swine dental pulp tissue, swine adipose tissue, or human dental pulp tissue is preferably used, because they are conveniently obtained and good sources for obtaining the immortalized stem cells with stable characteristics.

Also, the inserted genes are preferably at least 2 or more gene sets selected from the group consisting of two early genes from papillomavirus, telomerase reverse transcription enzyme (TERT), and bmi, because of preparing the immortalized stem cells with stable characteristics. Also, it is preferable that the early genes from papillomavirus are human papillomavirus E6 or human papillomavirus E7 due to expression efficiency.

It is more preferable to prepare DNA fragments including any one of the following gene sets; (a) human papillomavirus E7 and telomerase reverse transcriptase (TERT); (b) bmi and TERT; (c) bmi, human papillomavirus E6 and TERT; (d) human papillomavirus E6, human papillomavirus E7, and TERT; (e) bmi, human papillomavirus E6, human papillomavirus E7, and TERT and to use. As TERT, hTERT is preferably used due to its expression efficiency.

Among the genes described above, TERT is the gene coding an enzyme to elongate telomere sequences, which is shortened due to aging. Both of Human papillomavirus E6 and E7 are the early genes of human papillomavirus. E6 is known to have the capability of the reactivation of TERT and degradation of PDZ domains. bmi-1 is the polycomb group gene and is known to involved in the self replication of stem cells and the differentiation of stem cells.

By introducing the set of genes as described above, it is confirmed that the combination of genes may efficiently express and it makes cells immortalize.

Following are explanations of the procedures in the case of preparing the lentivirus vector and introducing two genes into the cell. In order to introduce the set (a), human papillomavirus E7 and TERT, for example, based on the sequence information, double-strand DNA of EcoRI/KoZal/E7/T2A4/hTERT/BamHI (SEQ ID NO: 1 in the sequence listing) is synthetized via a standard procedure. The acquired DNA fragment is cloned into the multi-cloning site of the lenti-plasmid vector (pLVSIN-CMV Neo) via the standard procedure to obtain the lenti-vector (E7T) to which the two genes as described above are introduced.

Similarly to this, when two different genes, for example, the set (b) is introduced, firstly, double strand DNA of EcoRI/KoZal/Bmi-1/T2A4/hTERT/BamHI including Bmi-1 (SEQ ID NO: 2 in the sequence listing) is synthesized via the standard procedure. The obtained DNA fragment is cloned into the multi-cloning sites of the lenti-plasmid vector (pLVSIN-CMV Neo) via the standard procedure to obtain the lenti-vector (BT) to which the two genes as described above are introduced.

Similarly, for introducing three genes, double-strand DNA of T2A3E6 is synthesized along with the sequence information, and then it is inserted between Bmi-1 and T2A4 in the lentivirus vector manufactured in the (v2) as described above. By this, the double-strand DNA of EcoRI/KoZal/Bmi-1/T2A3/E6/T2A4/hTERT/BamHI (SEQ ID NO: 3 in the sequence listing) is produced. Thus obtained DNA fragment is cloned into the multi-cloning site in the lenti-plasmid vector (pLVSIN-CMV Neo) by using the conventional procedure to obtain the lenti-vectors (BE6T) to which above mentioned three genes are incorporated.

Similarly, for introducing four genes, double-strand DNA of E6T2A3 is synthesized and inserted between Kozak sequence and E7 in the lentivirus vector manufactured in the (d) as described above. By this, EcoRI/KoZal/E6/T2A3/E7/T2A4/hTERT/BamHI (SEQ ID NO: 4 in the sequence listing) is manufactured. Thus, the obtained DNA fragment is cloned into the multi-cloning site in the lenti-plasmid vector (pLVSIN-CMV Neo) by using the conventional procedure to obtain the lenti-vector (E6E7T) to which above-mentioned three genes are incorporated. Synthesis of the DNA fragments described above may be conducted by outsourcing to a company providing DNA synthesis services.

For retrovirus, gene introduction is conducted by different procedures from those for the lenti virus vectors. Genes are introduced by co-transfection of vectors so as to contain each gene. For example, Kozak sequence (gccacc) is added into the upstream of the following five immortalizing genes: hTERT (SEQ ID NO: 5 in the sequence listing), human papillomavirus E7 (SEQ ID NO: 6 in the sequence listing), human papillomavirus E7 (SEQ ID NO: 7 in the sequence listing), pigTERT (SEQ ID NO: 8 in the sequence listing), and hBmi-1 (SEQ ID NO: 9 in the sequence listing)) by using the conventional procedure. Then, they are cloned into PmaC I-Hpa I site in pDON-5 NEO DNA (TaKaRa Code 3657) by using the conventional method to prepare Retrovirus plasmid vectors (pDON-5 Neo hTERT vector, pDON-5 Neo HPV16E6 vector, pDON-5 Neo HPV16E7 vector, pDON-5 Neo pHTERT vector, and pDON-5 Neo hBmi1 vector).

After that, E.coli is transformed by using 5 plasmid DNAs obtained as described above by using the conventional method, the obtained transformants are incubated in a CO₂ incubator to obtain the plasmid DNA with transfection grade.

Next, G3T-hi cells are inoculated at 5.5 - 6.5 × 10⁶ cells/dish in desired plates, and they are incubated in a 5 % CO₂ incubator at 37 °C for about 20 - 28 h. Then, desirable amount of a transfection reagent (for example, 0.3 - 0.5 mL of TransIT-293) is added to the culture. Subsequently, 3 plasmid vectors are chosen from 5 plasmid vectors obtained as described above for co-introduction with both of pGP and pE-Ampho (both of them are the vectors provided as the appendix of Retrovirus Packaging Kit Ampho). After that, they are further cultured under the same condition as that employed in the precedingly conducted culture for 40-56 h during the term genes are introduced into the cells.

### 1.2 Preparation of cells producing the vectors

The preparation of cells producing recombinant lentivirus vector is prepared at the same time as the procedure conducted as described above. As such cell strains, for example, there are mentioned as Lenti-X 293T (Clontech, code No. : 632180) and other commercially available cell lines. For the culture of Lenti-X293T, a medium supplemented with fetal bovine serum and antibiotics may be used. As the medium, there are mentioned as minimal essential medium (MEM) and Dulbecco's modified Eagle medium (DMEM) and the like. For example, it is preferable to use DMEM (produced by SIGMA, St. Louis, MO) supplemented with 5 - 15 % FBS (produced by HyClone) and 0.5 - 2 % of antibiotics (penicillin/streptomycin, produced by Gibco) from the view point of cell proliferation rate.

For example, DMEM is prepared by being supplemented with 10 % FBS and 1 % penicillin/streptomycin, and it is used as a basal medium. Herein below, in the specification, the medium is referred to as "293T basal medium".

For example, when Lenti-X 293T is used as the cell strain, the cell suspension including the cells at 1-5 × 10⁵ cells/mL is prepared, and 10 mL of the suspension is poured into 10 cm dishes. Then, the dishes are incubated in a 5 % CO₂ incubator for 24 h. After that, the cells in the dishes are passaged depending on the cell condition until use. When using the cells, firstly, the culture supernatant of the cells experiencing several passages are removed. Then, the cells are washed with PBS, and are detached from the bottom surface of the dish by using commercially available detachment reagent to be collected. The viable cell number of the desirable amount of the cell suspension diluted to 1/3 or 1/5 is counted with a hemacytometer by adding the desirable amount of trypan blue solution.

The cell suspension at 5 × 10⁵ cells/mL is prepared by adding the basal medium, and then, for example, the cell suspension is inoculated into dishes so as to be the desirable concentration, and incubated under the desirable conditions. For example, the cells are placed in the dishes having a diameter of 6 cm so as to 1 - 4 × 10⁶ cells/4mL, and are incubated in the 5 % CO₂ incubator at about 37 °C for about 24 hr. The culture supernatant is exchanged to fresh one, and then they are further cultured.

It is preferable to use G3T-hi cells (produced by TAKARA Bio), a high expression cell line of GnT-III as cells for retrovirus preparation, which is produced by introducing human N-acetylglucosaminyltransferase III (N-acetylglucosaminyltransferase III: GnT-III) with the hygromycin-resistant genes into the human kidney-derived cell line 293T (G418 resistant).

G3T-hi cells are designed to produce high titer recombinant virus promptly and transiently by co-introducing an expression vector harboring gag-pol and env genes, and recombinant retrovirus vector plasmid harboring objective genes with Retrovirus Packaging Kit Eco or Ampho (both are produced by Takara Bio, product code Nos.: 6160, 6161). Since G3T-hi cells are derived from 293T cells, it has an introduced T-antigen gene of SV40, thereby RNAs of a retrovirus are amplified to produce high-titer virus solutions. In general, the transient expression by using a Retrovirus Packaging Kit gives the solutions containing 10⁵-10⁷ cfu/mL viruses.

In G3T-hi cells, glycans on the cell surface are modified by GnT-III. Since sprouting retroviruses wear the host cell membrane, it is assumed that the glycan on the membrane proteins of the recombinant retroviruses obtained from the G3T-hi cells are modified by GnT-III. Due to the glycan modification, the retrovirus prepared with the G3T-hi cells has a high affinity to RetroNectin (recombinant human fibronectin filament). Therefore, the use of RetroNectin as a coating agent for the culture dish highly improves the efficiency of gene introduction into target cells. In particular, RetroNectin effectively functions for gene introduction, targeting into hematopoietic cells.

When using the G3T-hi cells, it is preferable to use DMEM containing glucose (4.5 g/L), L-glutamine (584 mg/L), supplemented with 10 % FBS and 1 % penicillin/streptomycin due to the production efficiency of the retroviruses.

### 1.3 Preparation of the virus vector

Hereinbelow, it is explained by using the lentivirus vector as an example.

As described above, the co-transfection is conducted by respectively adding the lentivirus vector plasmids prepared as described above into the dishes, wherein the cells are cultured as explained above. For such a co-transfection, commercially available packaging systems, for example, Lenti-X HTX packaging system (Clontech Laboratories Inc.) and the like may be used. Concrete procedures may be conducted according to manuals attached to them.

After co-transfection, the medium in the dish is replaced with fresh complete medium and incubated at about 37 °C for 24 to 28 hours. The virus vector is titered to decide the harvesting time point, and the culture supernatant including the virus vector is collected when the virus titer becomes maximal. For the titration of the virus vector, the commercially available convenient titration kit is used. As the examples of such kits, there are mentioned such as Lenti-X GoStix (Clontech Laboratories Inc.) and the like.

For example, the day after the medium change, the culture supernatant in the petri dish is collected by using the desired size of a syringe. The collected supernatant is filtrated to obtain the virus vectors. For example, the supernatant is taken out by using 10 mL of disposable syringe (Terumo Corporation), and then, it is filtrated by using a filter, for example, 0.45 µm of the filter (MILEX-HV, Millipore limited), attached to the syringe. By this, the virus vector solution is collected into a tube, for example, 15 mL of a tube.

Next, reagents included in the commercially available convenient titration kit are used to confirm the presence of the recombinant lentivirus vector. For example, the desirable volume of the virus vector solution, for example, 20 µL, is added to S of the Lenti-X GoStix, and Chase Buffer 1 is further added to the solution. Then, the solution is reacted at the desirable temperature and time period, for example, 10 minutes. The presence of the recombinant lentivirus vector is confirmed with/without the appearance of bands.

Cells harboring 4 plasmids (E7T, BT, BE6T, and E6E7T) obtained as described above are streaked on agar plates. Formed colonies on the agar are picked up for plating into the desirable medium, for example, 2 mL of LB medium containing antibiotics, and then they are vigorously cultured in a shaker at about 37 °C for 8 hours. Next, the desired amount of cultivated solution of the cells as described above, for example, about 100 µL, is added into LB medium, for example, 50 mL of LB medium, supplemented with different antibiotics, for example, ampicillin. Then, the medium is vigorously cultured in a shaker at about 37 °C for about 14 to 18 hours.

The amount of plasmid eluted with the commercially available kit, for example, MN NucleoBond Xtra Mid Kit (Clontech Laboratories Inc.) is measured. Together with this procedure, the plasmid is analyzed for confirming that the inserted DNA is mutually different using a desirable gel, for example, about 1 % agarose gel. As the marker for the gel electrophoresis, for example, supercoil DNA ladder, λ-Hind II digested DNA, and the like may be used. As described above, the virus vector is prepared.

### 2. Preparation for mammal stem cells

### 2.1 Preparation for the swine dental pulp stem cell

Swine jaw (lower jaw having teeth) from the swine of 5 to 6 months old and mesentery are obtained. According to the following procedure, swine dental pulp stem cells (herein below they are sometimes referred to as "SHED") are obtained from the swine teeth and the swine lower jaw.

Firstly, the swine teeth and the lower jaw are disinfected using an appropriate disinfection agent such as Isodine. Then, a crown of the tooth is cut in the horizontal direction by using, for example, a diamond point for a dentist and the like. Then, the dental pulp is collected from both of the dental crown and dental root portions treated as mentioned above by using, for example, a hand scaler for the dentist and the like.

Obtained dental pulps are chopped, for example, by using an ophthalmic knife to be suspended in a predetermined concentration of collagenase solution, for example, 1 to 3 mg/mL to stand for 1 hour in a single cell. Then, they are preliminary cultured in DMEM containing 10 % FBS, and 1 % Anti-Anti (Invitrogen, Carlsbad, CA) at 37 °C in a 5 % CO₂ incubator for obtaining the cells for passage.

Until the cell numbers are increased to sub-confluent, the medium is replaced 2 to 3 times per week. Then, the cells in the sub-confluent state are detached from the dish using a releasing agent such as Hepes solution containing 0.05 % trypsin. After that, the cells are collected by centrifugation under the desirable conditions such as at 1,500 rpm for 3 minutes at room temperature. The obtained cells are transferred into the fresh medium and conducted cell passage by using the desirable amount, for example, the entire volume.

### 2.2 Preparation for dental pulp stem cells from human exfoliated dens deciduous teeth

Exfoliated or extracted dens deciduous teeth are obtained from healthy children. These dens deciduous teeth are disinfected by using an appropriate disinfection agent, for example, Isodine solution, and then the dental pulp tissues are collected in the same as those for obtaining the swine dental pulp tissues. Obtained dental pulp tissues are digested in the solution including the desired concentrations of collagenase and dispase, for example, about 3 mg/mL of type I collagenase and about 4 mg/mL of dispase at the desired temperature for the desired time, for example, about 37 °C for about 1 hour. Next, the solution is filtrated by using, for example, 70 mm of a cell strainer (Falcon) to separate cells.

The filtrated cells are re-suspended, for example, in 4 mL of the above-mentioned medium, and spread on culture dishes for adherent cell culture with desirable diameter, for example, 6 cm. Desirable medium, for example, DMEM supplemented with about 10 % FCS, is added to the dishes and cultured for a desirable period, for example, 2 weeks in the incubator under the conditions of 5% CO₂, at 37 °C. Adherent cells formed colonies, the dental pulp stem cells, are treated with the desirable releasing agent, for example, about 0.2 mM EDTA containing about 0.05 % of trypsin for a desirable time period, for example, 5 minutes, at about 37 °C to collect the cells released from the dishes.

Next, the adherent cells collected as described above are spread, for example, in the dishes for the adherent cells (collagen-coat dish), and they are primarily cultured in an incubator under the conditions of, for example, 5% CO₂, at 37 °C to obtain primary cultured cells. When the cells become sub-confluent or confluent by macroscopic observation, the cells are treated the same as those described above by using the releasing agent to collect the cells in the dish.

After that, the primarily cultured cells are passaged by using the above-mentioned medium at the desirable concentration, for example, about 1 × 10⁴ cells/cm². The cells passaged 1 to 3 times are employed in experiments. As described above, human exfoliated dens deciduous dental pulp stem cells (SHED) may be obtained.

Depending on the necessity, the dental pulp stem cells from the swine dens deciduous dental pulp stem cells as described above, swine adipose stem cells and the dental pulp stem cells from the human exfoliated dens deciduous dental pulp stem cells may be poured into vials respectively, and may be cryopreserved -80 °C.

### 3. Preparation of gene-introduced cells

### 3.1 Gene introduction by the lentivirus vector

The swine dental pulp stem cells, the swine adipose stem cells, and the human dental pulp stem cells obtained as described above are cultured as mentioned above, respectively. When the cells reached about 70 to 90 % confluent, they are subjected to mycoplasma check, which is conducted with a commercially available kit. As such a kit, for example, MycoAlert Mycoplasma Detection Kit (Lonza, LT07-318) and the like may be used. Next, the lentivirus vector prepared as described above infects the cells respectively by using Polybrene reagent. Then, the cells are selected under the selection pressure of the reagent to obtain the strains stably expressing the target genes.

The culture supernatants of the cells cultured as described above are removed, and then the cells are washed, for example, with PBS (pH 7.4), respectively. After that, the cells are detached from the dishes using a desirable releasing agent, and then collected respectively. As the releasing agent, for example, StemPro (Registered trademark: GIBCO) may be used for the swine dens deciduous dental pulp stem cells and the swine adipose stem cells; and trypsin - EDTA and the like may be used for human dental pulp stem cells.

Obtained cells are counted according to the conventional method, and then they are spread into each well of the plate, for example, a 6-well plate (CORNING) with tissue culture treatment (T. C. treatment) so as to be about 1 × 10⁵ cells/well. They are incubated in the CO₂ incubator at about 37 °C for about 24 hours to confirm even distribution of the cells in the well.

After that, the culture supernatants are removed. The desired volume of the desired medium containing the desired concentration of Polybrene, for example, the desirable amount of DMEM containing about 8 µg/mL of Polybrene and about 10 % of FBS, is added for the dental pulp stem cell of the swine dens deciduous dental pulp stem cells at 750 µL/well. Next, the lentivirus vector solution described above is added into each cell, for example, at about 250 µL/well.

When the human dental pulp stem cell of the dens deciduous teeth is used, DMEM containing the same amount of Polybrene and FBS is added into the wells at a desirable amount, for example, about 1.2 mL/well, and then, the lentivirus vector solution is added to each well at the desirable amount, for example, about 400 µL/well.

Each plate to which the virus vectors are added as described above is centrifuged under the desirable conditions such as at about 1,000 × g for about 30 minutes, at about 32 °C for virus infection to the cells. After that, they are cultured under the desirable conditions such as at about 37 °C in a CO₂ incubator for about 4 to about 6 hours; and a desirable volume of the fresh culture medium, for example, about 1 mL/well, is added into each well. Then, they are cultured under the desirable conditions, for example, at 37 °C in the CO₂ incubator for about 24 hours to conduct the gene introduction.

### 3.2 Selection under the presence of the agents

The culture supernatants of the cultured stem cells (namely, the swine dental pulp stem cells, swine adipose stem cells, and human dental pulp stem cells), which are cultured as described above, are removed from each well of the culture plate. Then, the desirable concentration of the agents for selection, for example, selection medium containing either about 0.4 mg/mL or about 0.8 mg/mL of GENETICIN (G418, GIBCO) is added into each well in a desirable volume, for example, about 2 mL/well to exchange the culture medium. After that, the cells are cultured for a desirable period, for example, about 3 to 5 days exchanging the culture medium for selecting the gene-introduced cells.

A portion of the cultured cells is subjected to cloning, and the remains are successively cultured in the selection medium as pool cells. The cloning may be conducted as follows.

For example, the selection medium without antibiotics, for example, the medium without penicillin, streptomycin, and G418 is used for dilution of the cells to a desirable concentration, for example, either about 1 × 10³ cells/4 mL or about 5 × 10³ cells/4 mL. They are plated in each dish and are incubated at about 37 °C in the CO₂ incubator for about 24 hours. Formed colonies are marked from the rear side of the dish until they reach the desirable number, for example, about 100. Then, the culture supernatant in the well is removed, followed by washing the cultured cells with PBS. Cloning rings are set in the dishes and the cells in each colony which are detached by the releasing agent are respectively spread in each well, for example, those of a 48-well plate at a desirable amount, for example, about 1 mL.

### 3.3 Preparation of total RNA

In order to confirm the gene expression, total RNA is prepared with, for example, NucleoSpin RNA II (a kit provided by MACHEREY- NAGEL). It is preferable to use the buffers employed below, the ring filter, and the like included in the kit, and to perform the procedure according to the manual included in the kit due to obtaining total RNA with high purity.

Cell lysate is prepared by lysing a cell pellet containing the desirable number of cells, for example, about 5 × 10⁵ cells. The lysate is poured onto the ring filter with a purple color, and then, it is centrifuged under the desirable conditions such as at about 11,000 × g for about 1 minute. After the centrifugation, the filter was discarded, and then, the desirable amount of ethanol, for example, about 350 µL of about 70 % ethanol is added into the collection tube. The solution in the collection tube is mixed with pipetting in desirable numbers such as about 5 times.

Next, the desirable amount of the obtained solution, for example about 700 µL, is loaded on the ring column with a pale blue color set in the collection tube. Then, the tube is centrifuged under the desirable conditions such as about 11,000 × g for about 30 seconds to bind RNA. After the centrifugation, the column is set in the new collection tube. Then, the desirable reagent such as about 350 µL of MDB is added into the tube, and centrifuged under the desirable conditions such as at about 11,000 × g for about 1 minute for desalination.

After the centrifugation, the DNA reaction mixture is prepared by mixing lightly, for example, the desirable amount of rDNase, for example, about 10 µL, and the desirable amount of DNase reaction buffer, for example, about 90 µL. The desirable amount of the mixture, for example, about 95 µL is added to the column, and incubated under the desirable conditions such as at room temperature for about 15 minutes to digest DNA therein. Subsequently, the desirable amount, for example, 200 µL of buffer RA2 is added to the column and they are centrifuged under the desirable conditions such as about 11,000 × g at about 30 seconds to wash out. Subsequently, the column is set in the new collection tube, and the desirable amount such as about 700 µL of buffer RA3 is added to the column for further centrifugation under the desirable conditions such as about 11,000 × g for about 30 seconds.

Subsequently, the eluted solution in the collection tube is discarded, and the desirable amount, for example, about 250 µL of the buffer RA3 is again added to the column for the centrifugation under the desirable condition, for example, at about 11,000 × g for about 2 minutes. Then, a silica membrane of the column is air-dried. The column is set in the desirable size such as about 1.5 mL of the collection tube, and the desirable amount of RNase-free water, for example, about 60 µL, is added to the column. Then, the column is centrifuged under the desirable conditions such as about 11,000 × g for about 1 minute to obtain the total RNA sample.

### 3.4 Reverse transcription

The total RNA sample obtained as described above is subjected to reverse transcription. Subsequently, it is subjected to real-time PCR to obtain PCR products. In order to conduct the reverse transcription, for example, a commercially available kit such as PrimeScript RT reagent Kit (Perfect Real-time, TaKaRa Bio) is used and reverse transcription is conducted according to the manual attached thereof.

The real-time PCR is conducted by using the commercially available kit, for example, SYBR Premix Ex Taq and the like. Also, as the standard genes used here, there are mentioned, for example, swine β-actin, human β-actin and the like. As primers, there are mentioned, for example, those listed in the sequence Nos. 10 to 15 are prepared for use.

Firstly, the desirable amount, for example, Premix for real-time PCR is prepared by mixing, for example, about 350 µL of SYBR Premix Ex Taq II (x2), about 28 µL of Primer mix (about 10 µL), and about 266 µL of redistilled water. Next, the desirable amount of Premix, for example, about 23 µL is poured into each tube for real-time PCR, and then about 2 µL of the template cDNA is added into each tube. Then, real-time PCR is conducted under the desirable conditions, for example, at about 95 °C for about 30 seconds, about 40 cycles (at about 95 °C for about 5 seconds, subsequently at about 60 °C for about 30 seconds). Subsequently, amplified products are denatured under the desirable conditions, for example, at about 95 °C for about 15 seconds, at about 60 °C for about 30 seconds, and at about 95 °C for about 15 seconds to obtain a melting curve. For the human dental pulp stem cells, the same procedures are conducted.

From the standard curve prepared as described above, the expression levels of the induced genes by using each virus vector are obtained. By calibrating with internal standard genes, accurate results are obtained.

In each gene-introduced stem cell, it is confirmed that the introduced genes are expressed. By the confirmation, the introduction efficiency of the genes by using each plasmid is obtained. Next, the gene-introduced cells (hereinbelow, they are sometimes referred to as "introduced-gene stably expressing cell population" or "pool cells".) are subjected to single cell cloning.

Firstly, the pool cells are plated in the desirable size dish with the desirable numbers, for example, into the dish having 60 mm diameter with about 1 × 10³ cells/dish, and they are cultured in a CO₂ incubator at the desirable temperature and time period, for example, at 37 °C for 24 hours. After that, the culture thereof is conducted by exchanging the growth medium containing the selection reagents described above, and further continued to form colonies. Every formed colony is individually detached using both the cloning ring and the releasing agent, and then they are spread on a desirable plate, for example, a 24-well plate.

After growing the spread cells, they are grown in the culture dishes and the like to culture in larger scale. By this, the cells which express the introduced-gene stably are obtained as single clones. Next, the obtained clone is cultured for growth as described above to obtain total mRNA. The obtained total RNA is used as the template for reverse transcription by using a desirable kit, for example, PrimeScript RT reagent Kit to obtain template cDNA.

After that, real-time PCR is conducted similarly to those described above; for example, by using cDNA (the reverse transcription products) as the template, SYBR Premix Ex TaqII (Tli RNaseH Plus) as described above, and the primers (specific primers for the transduced genes or the internal standard genes respectively, for example, the primers shown as SEQ ID NO.: 10 to 15).

Ct value, which is calculated from the secondary differentiation curve of the real-time PCR, is plotted on the X-axis, and the relative total RNA value is plotted on the Y-axis to prepare the standard curve to determine the amounts of each gene expression. The relative expression values of the introduced genes expressed in the swine dental pulp stem cell or those derived from human dental pulp stem cells are obtained when the expression amounts thereof on pool cells are set as 1. By this, it is confirmed that whether entire introduced genes are expressed or not, and their expression amounts, which are used for the selection of objective clones.

As described above, both the immortalized stem cell population and the clones therefrom, which are cloned from the immortalized stem cells therefrom, are obtained.

A certain transformant is selected from the obtained transformants, for example, ampicillin is used to obtain ampicillin -resistant (herein below referred to as "Amp+") transformant. Then, the Amp+ transformant is purified, and identified by analyzing restricted enzyme sites. Next, the recombinant adenoviruses are digested with Pad to obtain their fragments, which are used to transfect to HEK293 cells for amplification thereof; and then the viruses described above is titrated. According to the conventional method, the virus is purified and transfected to the target cells, SHED-P.

The virus-transfected HEK293 cell population is stained with FITC according to the conventional method to detect STRO-1-positive cells among the population by using the flow cytometer. STRO-1 is considered as the maker of MSCs having the pluripotent capability in bone marrow, and it is an index of cell immortalization. By following the protocol as described above, the immortalized stem cells derived from dental pulp are obtained.

Next, the obtained immortalized stem cells are cultured in the basal medium described above, for example, DMEM supplemented with 10 % FBS under the conditions of 5 % CO₂ and 37 °C for 24 - 48 hours to obtain the conditioned medium. The conditioned medium is collected by using a pipette such as the Komagome pipette.

The immortalized stem cells secret growth factors such as Insulin-like growth factors (IGFBPs), Vascular Endothelial Growth Factors (VEGFs), Tissue Metalloproteinase Proteins (TIMPs), Hepatocyte Growth Factors (HGFs), and the like into the culture medium. Here, the term, "growth factor", is a generic name of polypeptides to promote cell division, to alter the cell morphology, or to enlarge the cells. The factors vary depending on the origin of the production cells. They are classified into groups such as Epidermal growth factor (EGF), fibroblast growth factor (FGF), nerve growth factor (NGF), and tumor growth factor (TGF), and the like.

Furthermore, the receptors on the cell membrane of each cell have tyrosine kinase activity. Upon binding growth factors, they phosphorylate tyrosine residues in proteins, which is a trigger of cell proliferation, differentiation, and the like. Some examples that the growth factors function as mesoderm inducers during ontogeny are known. Also, some examples that lymphokines, which regulates immune system, function as the mesoderm inducers during ontogeny are known. These growth factors may be determined by using ELISA or microarray method, both of which are publicly known.

IGFBPs are polypeptides of which sequence is highly similar to that of insulin, and cause reactions such as mitosis induction similar to those of insulin in cell culture. They are also known to affect neuronal growth. VEGF is a member protein of glycoprotein group relating to vasculogenesis for forming new vascular in the area without blood vessels; and angiogenesis for forming blood vessels by branching and elongating from already existing blood vessels in the embryonic stage. HGF has a variety of bioactivities against hepatocytes and other cells such as promoting cell proliferation, or cytotropism, anti-apoptosis (cell death), induction of morphogenesis, angiogenesis, protection and regeneration of other tissues and organs.

The conditioned medium containing the growth factor is obtained by culturing each stem cell described above, for example, in DMEM supplemented with 15 % FCS at 37 °C for the desirable time period. The conditioned medium of the stem cells contains not only IGFBPs, VEGF, and HGF, but also other many proteins.

15 mL portion of the obtained conditioned medium are placed into an Amicon Ultra Centrifugal Filter Units-10K (Millipore) and is concentrated to 200 µL by centrifugation at 4,000 × g for about 60 minutes. Next, sterilized PBS equal volume to the conditioned medium is added to the tube, and then it is centrifuged at 4,000 × g for about 60 minutes again to replace the solvent to PBS. 200 µL portion of the obtained solution is collected into a micro test tube, which is the concentrated stem cell conditioned medium.

Instead of the method using the Amicon, ethanol precipitation may be used for concentration. For example, 45 mL of 100 % ethanol is added to 5 mL of the conditioned medium, which is mixed and stood at -20 °C for 60 minutes. After that, the mixture is centrifuged at 15,000 × g at 4 °C for 15 minutes, and then the supernatant is discarded.

Next, for example, add 10 mL of 90% ethanol to the residue in the tube, and they are mixed well, and it is centrifuged again at 15,000 × g for 5 minutes at 4 °C. The supernatant is removed, and the resulting pellet is dissolved in, for example, 500 µL of sterilized water. After dissolution, the entire volume thereof is collected into the micro-test tube, which is the concentrated stem cell conditioned medium.

By lyophilizing the conditioned medium obtained as described above according to a conventional method, a composition of particle powder 13 for prepared at use may be obtained.

### EXAMPLE

### (Example 1) Production of immortalized SHED and preparation of conditioned medium (1) Reagents

### (1-1) Reagents for plasmid extraction

Kanamycin (Kan), ampicillin (Amp), LB liquid medium and LB agar medium, glycogen, agarose, sterilized water, ammonium acetate, sodium acetate, sodium dodecyl sulfate and RNase A are used. 50 mg/mL of kanamycin (Kan) and ampicillin (Amp) are prepared and stored at -20 °C as stock solutions. Glycogen solution is prepared at 20 mg/mL. 10 mg/mL RNase A solution is prepared and stored at -20 °C. 10 M (saturated) ammonium acetate (NH₄OAc) and 3 M sodium acetate (NaOAc; pH 5.2) are prepared.

### (1-2) Restriction enzyme and so on

E. coli Competent Cells (Supercharge EZ10 Electrocompetent Cells, product code 636756), SwaI (product code 1111A, SmiI is the equivalent), XhoI (product code 1094A), T4 DNA Ligase (product code 2011A) NucleoBond Xtra Midi (product code 740410.10/.50/.100), NucleoSpin Plasmid (product code 740588 10/50/250) are all purchased from Takara Bio Inc., and PacI is purchased from New England Biolabs.

### (1-3) Buffers and so on

1×TE Buffer (10 mM Tris-HCl [pH 8.0] containing 1 mM EDTA) and phenol: chloroform: isoamyl alcohol (25:24:1, hereinafter referred to as "PCI mixture") saturated with 100 mM Tris-HCl (pH 8.0) are prepared. Ethanol is used at 100% and 70%. The following buffers 1-4 are prepared for purification of pAdeno-X plasmid DNA for use in mini-scale recombination.

Buffer 1: 25 mM Tris-HCl containing both 10 mM EDTA and 50 mM Glucose (pH 8.0) (stored at 4 °C after autoclave)
Buffer 2: 0.2 M NaOH containing 1 % SDS (prepared just before use, sealed up, and stored at room temperature)
Buffer 3: 5 M KOAc (stored at 4 °C after autoclave)
Buffer 4: 10 mM Tris-HCl containing 1 mM EDTA and 20 µg/mL RNase (add RNase just before use, sealed up, and stored at - 20 °C)

### (2) Purification of Adenovirus and reagents for β-gal assay

Human HEK293 cells (ATCC #CRL1573) transformed with human Type 5 adenovirus is used. HEK293 cells are cultured in a complete medium. The composition of the complete medium is DMEM (Dulbecco's Modified Eagle's Medium, basic medium) supplemented with 100 units/mL sodium penicillin G, 100 µg/mL streptomycin, 4 mM glutamine, and 10% FBS. Penicillin G sodium solution is prepared at 10,000 units/mL and streptomycin sulfate solution at 10,000 µg/mL respectively and stored as stock solutions. For cell culture, 60 mm plates, 100 mm plates, 6-well plates, T75 and T175 flasks are used.

Trypsin-EDTA (product code CC-5012) was purchased from Takara Bio Inc. Phosphate buffered saline (PBS, without Ca²⁺ and Mg²⁺) and Dulbecco's phosphate buffered saline (DPBS, with Ca²⁺ and Mg²⁺) are prepared. In addition, 0.33% neutral red staining solution and 0.4% trypan blue staining solution are used. X-Gal (5-bromo-4-chloro-3-indolyl-β-D-galactopyranoside (25 mg/mL)) in dimethylformamide (DMF) solution for β-gal assay is stored light-shielded at -20 °C. Luminescent β-gal Detection Kit II (Takara Bio, product code CC-5012) is used.

### (3) Preliminary test

### (3-1) Construction of recombinant Adenovirus containing lacZ (pAdeno-X-lacZ)

HEK293 cells are thawed and removed DMSO from the medium, and then are resuspended in 10 mL of the complete medium described above. The entire volume is transferred into a 100 mm diameter of a culture plate. After HEK293 cells are attached, the culture medium is removed, and the cells are washed once with sterilized PBS. 1 mL of trypsin-EDTA solution is added there for trypsin treatment and reacted for approximately 2 min. Next, 10 mL of complete medium is added to stop the reaction of trypsin and then gently suspended. Viable counts are performed and 10⁵ cells are transferred to a 100 mm diameter plate containing 10 mL of culture medium and spread evenly.

Using pShuttle2-lacZ (a positive control vector contained in Adeno-X Expression System 1) and Adeno-X Viral DNA (PI-Sce I and I-Ceu I digested) included in the kit, a recombinant adenovirus containing lacZ is constructed according to the protocol provided in the kit. The target cells, SHED, are infected by the adeno virus and assayed for β-galactosidase expression to confirm that the vector is constructed.

### (3-2) Construction of recombinant pShuttle 2 plasmid

Prior to the construction of the recombinant pShuttle 2 Vector (Herein below, it is referred to as "rpShuttle 2 Vector"), DH5α E. coli are transformed with both of the pShuttle 2 Vector and pShuttle 2-lacZ Vector included in the kit. Transformed bacteria are selected from the colonies on an LB agar plate containing 50 µg/mL Kanamycin (Herein below, it is referred to as "LB/Kan"). The bacteria obtained from a single colony are streaked onto new LB/Kan and incubated overnight at 37 °C.

Next, hTERT, bmi-1, HPV-E6, and HPV-E7 are cloned into pShuttle 2 according to the following procedures. pShuttle 2 Vector is digested with restriction enzymes appropriate for these genes. Subsequently, the pShuttle 2 Vector Information Packet (PT3416-5) provided with the above kit is referred to determine the multicloning sites matching the DNA to be inserted. The plasmid described above, which is treated with the restriction enzyme, is treated with alkaline phosphatase and then purified.

Target DNA fragments are prepared and purified according to the conventional method. The above restriction enzyme-digested vector is ligated with the above gene fragment; and DH5α cells (competent cells) were transformed with the ligation product. A portion of the above competent cells was taken and transformed with the control vector pShuttle2-lacZ Vector included in the kit and used as a positive control.

The mixture containing transformed E. coli is inoculated onto LB/Kan agar plates and transformants (colonies) resistant to kanamycin (Kanr) are selected. 5-10 of Kan-resistant clones are selected, transferred into a small amount of liquid medium, and expanded. After confirming that these clones harbored the rpShuttle 2 Vector, they are incubated overnight. The constructed plasmid DNA is then purified according to the conventional method using a commercially available silica adsorption column.

The plasmid DNA is treated with restriction enzymes and subjected to 1% agarose gel electrophoresis to identify the recombinant plasmid of interest. By sequencing, the direction and insertion site of the inserted fragment are confirmed, and positive clones are identified. Recombinant pShuttle 2 plasmid DNA ("rpShuttle2 plasmid DNA") is directly transfected into the target cells, and then they are subjected to Western blotting for preliminarily check of the expression of the target protein.

### (3-3) Double digestion of the rpShuttle2 plasmid DNA with PI-Sce I/I-Ceu I

The expression cassette of the transgene introduced into the plasmid is excised from the rpShuttle2 plasmid DNA prepared as described above with both of PI-Sce I and I-Ceu I. The expression cassette is incorporated into Adeno-X Viral DNA according to the in vitro ligation method described in the protocol provided with the kit. 30 µl of PI-Sce I/I-Ceu I double digestion reagent of rpShuttle 2 plasmid DNA is prepared, and added into 1.5 mL of micro centrifuge tube containing the reagents listed in Table 1 below.

**[Table 1]**

| Reagents etc. | Amount of reagent (µL) | |
|---|---|---|
| | Tube 1 | Tube 2 (lacZ control) |
| Sterilized water | 19.5 | 19.5 |
| 10 × double digention reagent | 3.0 | 3.0 |
| rpShuttle2 plasmid DNA (500 ng/µL) | 2.0 | - |
| pShuttle2-lacZ plasmid (500 ng/µL) | - | 2.0 |
| PI-Sce I (1 unit/µL) | 2.0 | 2.0 |
| I-Ceu I (5 unit/µL) | 0.5 | 0.5 |
| 10 × BSA | 3.0 | 3.0 |

Next, after thorough mixing, the reaction solution in the microcentrifuge tube is centrifuged in short time, and then incubated at 37 °C for 3 hours. The reaction solution (5 µL) after the above double digestion is subjected to the electrophoresis on a 1% agarose/EtBr gel along with the 1 kb ladder (DNA size marker).

### (3-4) Extraction with Phenol: chloroform: isoamyl alcohol

In a centrifuge tube, 70 µL of 1 × TE Buffer (pH 8.0) and 100 µL of PCI mixture are added to the remainder of the double digestion solution described above (25 µL) and thoroughly stirred by vortex mixer. The mixture is then centrifuged at 14,000 rpm for 5 minutes at 4 °C using the microcentrifuge, and the aqueous layer was transferred to a clean 1.5 mL microcentrifuge tube. 400 µL of 95 % ethanol, 25 µL of 10M ammonium acetate, and 1 µL of glycogen (20 mg/mL) are added into the tube, and they are thoroughly stirred in the tube by the vortex mixer.

Next, the tube is centrifuged at 14,000 rpm for 5 minutes at 4 °C, and the supernatant in the microtube is removed by aspiration to obtain a pellet. 300 µL of 70% ethanol is poured onto the pellet in the tube, and then the tube is centrifuged at 14,000 rpm for 2 minutes at room temperature. The supernatant in the tube is carefully removed by aspiration to obtain the pellet, which is air-dried at room temperature for approximately 15 minutes.

After the pellet is dried, it is dissolved by using 10 µL of sterilized 1 × TE Buffer (pH 8.0) and stored at -20 °C until use.

### (4) Construction of recombinant Adeno-X plasmid DNA

### (4-1) Subcloning of an expression cassette to Adeno-X virus genome

The reagents listed in Table 2 below are sequentially placed in 1.5-mL sterilized microcentrifuge tubes. Then, they are mixed gently in the tube, subjected to gentle centrifugation, and incubated at 16 °C overnight.

**[Table 2]**

| Reagents etc. | | Amount (µL) | |
|---|---|---|---|
| pShuttle2 plasmid DNA digested by PI-Sce I/I-Ceu I | | 2.0 | |
| pShuttle 2-lacZ plasmid DNA digested by PI-Sce I/I-Ceu I | | - | |
| Sterilized water | | 3.0 | |
| 10× DNA Ligation Buffer | | 1.0 | |
| Adeno-X Viral DNA (250 ng/µL) | | 3.0 | |
| DNA Ligase (1 unit/µL) | | 1.0 | |
| | Total | 10.0 | |

To each sample, 90 µL of 1 × TE Buffer (pH 8.0) and 100 µL of PCI mixture are added, gently stirred by vortex mixer. Then the tube is centrifuged at 14,000 rpm for 5 min at 4 °C, and after that the aqueous layer is transferred into the clean 1.5 mL microcentrifuge tube, to which 400 µL of 95% ethanol, 25 µL of 10 M ammonium acetate, and 1 µL of glycogen (20 mg/mL) are added. Then, the tube is gently stirred by the vortex mixer.

The tube is centrifuged at 14,000 rpm for 5 minutes at 4 °C, and then the supernatant is removed by aspiration to obtain the pellet. The following ethanol precipitation was performed as in (3-4) above.

After the pellet is dried, it is dissolved in 15 µL of sterilized deionized water.

### (4-2) Digestion of recombinant Adeno-X plasmid DNA with SwaI

The digestive solutions shown in Table 3 below are prepared and added to each sample in centrifuge tubes and incubated at 25 °C for 2 hours.

**[Table 3]**

| Reagents etc. | | Amount (µL) | |
|---|---|---|---|
| Ligation product | | 15 | |
| 10 × Swa I Digestion Buffer | | 2.0 | |
| 10 × BSA | | 2.0 | |
| Swa 1(10 units/µL) | | 1.0 | |
| | Total | 20.0 | |

Both 80 µL of 1 × TE Buffer (pH 8.0) and 100 µL of PCI mixture are added into each sample in the tube, and then they are gently stirred with the vortex mixer. The samples are centrifuged in microcentrifuge tubes at 14,000 rpm for 5 minutes at 4 °C. The following ethanol precipitation operations were performed as described above (3-4), and the pellet lysate was stored at -20 °C until use.

### (4-3) Confirmation of E.coli transformed with the recombinant Adeno-X plasmid DNA

A competent cell for electroporation, Supercharge EZ10Electrocompetent Cell (Product code 636756) is transformed by the product digested by SwaI as described in (4-2). The transformation mixture is inoculated onto agar plates with ampicillin (final concentration 100 µg/mL) added to LB medium ("LB/Amp agar plates") and incubated at 37 °C overnight. Approximately 10⁶ colonies were obtained as ampicillin-resistant (Ampr) transformants. The obtained colonies are checked with the Adeno-X System PCR Screening Primer Set provided with the product.

Bacteria from a single colony are inoculated into 5 mL of fresh LB/Amp liquid medium and incubated overnight. Next day, Adeno-X plasmid DNA is purified according to the mini-scale method described below.

### (5) Preparation of the mini-scale of the recombinant Adeno-X plasmid DNA

5 mL of the culture medium in log phase in a tube is centrifuged at 14,000 rpm for 30 seconds and the supernatant is removed. The pellet is centrifuged again at 10,000 rpm for 1 minute, and the supernatant is removed by using a micropipette. 150 µL of Buffer 1 is added into the tube, and gently pipetted to resuspend the cells. 150 µL of Buffer 2 is added into the call suspension, mixed by gentle inversion, and then left on ice for 5 minutes. 150 µL of Buffer 3 is added into the cooled cell suspension, subsequently mixed again by inversion, and then left on ice for 5 minutes.

The cell suspension is centrifuged at 14,000 rpm for 5 minutes at 4 °C, and the clear supernatant is transferred into a clean 1.5-mL centrifuge tube. 450 µL of PCI mixture is added into the supernatant in the tube, and then the mixture is stirred by inversion. The mixture is then centrifuged at 14,000 rpm for 5 minutes at 4 °C, and the aqueous layer is transferred into another clean 1.5-mL microcentrifuge tube.

The following ethanol precipitation is performed as the same as that described in (4-1) above, and the pellet lysate is stored at -20 °C until use. The target rDNA is identified by using restriction enzyme analysis and PCR as described below.

### (6) Restriction enzyme position analysis of the obtained rAdeno-X plasmid DNA

The analysis is performed with both PI-SceI and I-CeuI. The reagents listed in Table 4 below are placed in a 1.5-mL sterilized microcentrifuge tube, and then 30 µL of PI-SceI/I-CeuI double digestion reaction solution is added. Then the mixture is thoroughly stirred, and gently rotated to collect the contents.

**[Table 4]**

| | Reagents etc. | Amount (µL) | |
|---|---|---|---|
| Sterilized water | | | 19.5 |
| 10 × double digestion reagent | | | 3.0 |
| rpAdeno-X DNA (500 ng/µl) (500 ng/µL) | | | 2.0 |
| pShuttle 2-lacZ plasmid (500 ng/µL) | | | - |
| PI-SceI (1 unit/µL) | | | 2.0 |
| I-CeuI (5 unit/µL) | | | 0.5 |
| 10 × BSA | | | 3.0 |
| | Total | | 30.0 |

Incubation thereof is performed at 37 °C for 3 hours for restriction enzyme treatment. After this treatment, the reaction solution is electrophoresed on a 1% agarose/EtBr gel.

### (7) Production of recombinant adenovirus

### (7-1) Preparation of rAdeno-X plasmid DNA for the transfection of HEK293 cells

The reagents and others listed in Table 5 below are mixed in the 1.5-mL sterilized centrifuge tube, and softly centrifuged in the microcentrifuge. The tube is then incubated at 37 °C for 2 hours for Pac I restriction enzyme treatment of rAdeno-X plasmid DNA.

**Table 5]**

| | Reagents etc. | Amount (µL) | |
|---|---|---|---|
| Sterilized water | | 20 | |
| pAdeno-X plasmid DNA (500 ng/µL) | | 10 | |
| 10 × Pad Digestion Buffer | | 4 | |
| 10 × BSA | | 4 | |
| PacI (10 units/µL) | | 2 | |
| | Total | 40 | |

60 µL of 1 × TE Buffer (pH 8.0) and 100 µL of PCI mixture are added into the tube. Then, the tube is gently stirred by a vortex and centrifuged at 14,000 rpm for 5 minutes at 4 °C in the micro centrifuge. The aqueous layer is carefully transferred to a clean 1.5-mL sterilized centrifuge tube.

The following ethanol precipitation is performed as the same as those described in (3-4) above, and the pellet solution is stored at - 20 °C until use. (7-2) Transfection to HEK293 cells with Adeno-X plasmid DNA digested by Pad
24 hours prior to the transfection of the plasmid DNA into HEK293 cells are spread at a cell number of 1-2 × 10⁶ (approximately 100 cells/mm²) per 60 mm diameter culture plate, and then incubated at 37 °C in the presence of 5 % CO₂.

The cells in each culture plate are transfected with 10 µL of Pac I-digested Adeno-X plasmid DNA, and Adeno-X DNA is introduced into HEK293 cells according to a standard transfection method (CalPhos Mammalian Transfection Kit, product code 631312, Takara Bio). From the next day of the transfection, we checked whether CPE (cytopathic effect) occurred. After 1 week, we were detached the cells adhering to the bottom and side surfaces of the culture plate by gentle agitation. The obtained cell suspension is transferred to the 15-mL sterilized conical centrifuge tube and then the tube is centrifuged at 1,500 × g for 5 minutes at room temperature.

The resulting precipitate is suspended in 500 µL of sterilized PBS. The freeze-thaw procedure is repeated three times by freezing the lysate in dry ice/ethanol and thawing it in a 37 °C thermostatic chamber to obtain a lysate with sufficiently thawed cells. The lysate is then lightly centrifuged to remove suspended solids, and the supernatant is transferred to another sterilized tube for immediate use. The portion not which is not used immediately is stored at -20 °C. 250 µL of the lysate is added to the cultured cells in the 60-mm plate, and the culture is continued under the same condition. The anti-Hexon antibody contained in the Adeno-X Rapid Titer Kit (product code 631028, Takara Bio Inc.) is used to measure adenovirus titer according to the instruction manual attached to this kit (PT3651-1).

### (7-3) Amplification of the viruses for the preparation of viruses with high-titer

Approximately 24 hours before starting this titer assay, HEK293 cells are inoculated into T75 flasks and then cultured overnight at 37 °C in the presence of 5 % CO₂ to ensure that they were 50-70 % confluent.

The next day of the culture start, the medium in the flask is replaced with a fresh medium containing the virus, and the cells were infected with the virus at MOI=10. After 90 minutes of incubation at 37 °C in the presence of 5 % CO₂, the flasks are taken out, and 10 mL of medium is added to the flasks.

After 3-4 days of incubation at 37 °C under 5 % CO₂ condition, CPE is checked. When 50 % of the cells are detached, the released cell suspension is prepared as described above and transferred into a 15-mL sterilized conical centrifuge tube. Cells are subjected to freeze-thaw by the same procedure as described above to have the lysate, using the Adeno-X Rapid Titer Kit (product code 631028) to obtain the titer of 10⁷ PFU/mL. In order to confirm that the packaged adenovirus genome has a copy of the transcription unit, of which form is functional or not, the lysate is subjected to Westen blot analysis.

### (8) Adenovirus infection to the target cells

### (8-1) Infection to the target cells

Prior to 24 hours before infection, 1 × 10⁶ SHEDs are inoculated onto 6-well plates. Next day of the inoculation start, the medium in the plate is removed, and then 1.0 mL of medium containing the virus is added to the center of each plate. The solution is spread evenly throughout on the monolayer formed by SHED.

SHED is incubated at 37 °C and in the presence of 5 % CO₂ for 4 hours to be infected by the viruses, and then fresh medium is added. The plate is further incubated at 37 °C in the presence of 5 % CO₂. The expressions of the transgenes are time-dependently analyzed 24 to 48 hours after infection.

### (8-2) Analysis of β-galactosidase expression in the infected cells

Expression of β-galactosidase in the adherent cells being infected by Adeno-X-lacZ is assayed by using Luminescent β-gal Detection Kit II (product code 631712, Clontech).

### 2. Production of retrovirus vectors

### (1) Reagents and the like

G3T-hi cells (Takara Bio, product code 6163), TransIT-293 (Transfection reagent, Takara Bio, product code MIR2704), Retrovirus Packaging Kit Ampho (Takara Bio, product code 6161), Retrovirus Titer Set (for Real Time PCR) (Takara Bio, product code 6166), One Step SYBR PrimeScript RT-PCR Kit (Perfect Real Time, Takara Bio, product code RR066A) are used. Also real-time PCR apparatus, Thermal Cycler Dice Real System (Takara Bio, Model TP800) is used.

### (2) Production of plasdmids

pDON-5 Neo (Takara Bio, catalog No. 3657) is used to produce retrovirus plasmids for the preparation of retrovirus vectors. Kozak sequence (gccacc) was added upstream of the start codon of the immortalized genes, TERT (from human or swine, herein below they are abbreviated as "hTERT" or "pTERT", respectively.), two human papillomaviruses E6 and E7, and hBmi-1 by using a conventional method.

By cloning 5 immortalized genes with Kozak sequence as described above into the PmacI-HpaI site of the pDON-5 Neo DNA, 10 mL of recombinant retrovirus vectors containing the following plasmids are prepared respectively: pDON-5 Neo hTERT Vector (SYN5587-1-4), pDON-5 Neo HPV16E6 Vector (SYN5587-2 -10), pDON-5 Neo HPV16 E7 Vector (SYN5587-3-7), pDON-5 Neo pigTERT Vector (SYN5587-4-9), or pDON-5 Neo hBmil Vector (SYN5587-5-1)-containing recombinant retroviral vector ( Amphotropic Envelope).

The inserted gene sequence of the obtained plasmid DNA as described above is confirmed by single-strand analysis. Using the plasmid DNA, E. coli is transformed to obtain transformants. After culturing the transformants, the plasmid DNA (about 50 µg) of the transformation grade was purified according to the conventional method, and the plasmid DNA solution is obtained as a DNA solution form wherein DNA is dissolved in the sterilized water.

### (3) Production of the recombinant retrovirus vector

6 × 10⁶ G3T-hi cells per dish are inoculated onto 5 tissue culture dishes (100 mm diameter) and cultured at 37 °C for about 24 hours. 3 plasmids for retrovirus production (Any one of the vectors selected from the group consisting of pDON-5 Neo hTERT vector, pDON-5 Neo HPV16E6 vector, pDON-5 Neo HPV16E7 vector, pDON-5 Neo pigTERT vector, and pDON-5 Neo hBmi1 vector, pGP and pE-Ampho provided with the Retrovirus Packaging Kit Ampho) per dish are co-transfected to the cells in the dish by using a transfection reagent and further cultured at 37 °C for 48 hours.

After the termination of the culture, the culture supernatant containing the retrovirus vectors is collected, and sterilized by filtration through a 0.45 µm filter (Millipore). The obtained filtrate is dispensed into sterilized tubes as 1 mL of aliquots.

### (4) Calculation of the titer of the recombinant retrovirus vector

The titer of the recombinant retrovirus vector is quantified using the Retrovirus Titer Set (for Real-Time PCR) and the One Step SYBR PrimeScript RT-PCR Kit (Perfect Real Time) according to the instructions provided with the kit. Tables 6 and 7 below show the reaction conditions for template processing and real-time PCR for the titeration of the retrovirus vectors.
Table 6 below shows the composition of the reaction solution used for the template treatment (treatment of the template with Dnase I) for the titration of retroviral vectors. Table 7 below shows the reaction conditions. That is, the solutions shown in Table 6 are incubated at 37 °C for 30 minutes. Then, the incubation temperature is increased to 70 °C, held at 70 °C for 10 minutes, and subsequently rapidly cooled to 4 °C.

**[Table 6]**

| Dnase I treatment | |
|---|---|
| Reagents | Used amount/reaction |
| Recombinant retrovirus vector solution | 12.5 µL |
| 10 × DNase I buffer | 2.5 µL |
| DNase 15 (5 U/µL) | 2.0 µL |
| RNase Inhibitor (40 U/µL) | 0.5 µL |
| RNase Free dHzO | 7.5 µL |
| Total | 25.0 µL |

Real-time PCR is performed under the following reaction conditions by using the solutions listed in Table 7 below. In order to create a dissociation curve, 40 cycles of (s1) 5 min at 42 °C, (s2) 10 sec at 95 °C, and then (s3) 5 sec at 95 °C and 30 sec at 60 °C are performed.

**[Table 7]**

| Reagents | Used amount/reaction |
|---|---|
| 2 × One Step SYBR RT-PCR buffer III | 12.5 µL |
| TaKaRa ExTaq HS (5 U/µL) | 0.5 µL |
| PrimeScript RT Enzyme Mix II | 0.5 µL |
| Forward Titer Primer FRT-1 (10 pmol/µL) | 0.5 µL |
| Reverse Titer Primer FRT-1 (10 pmol/µL) | 0.5 µL |
| RNase Free dH₂O | 8.5 µL |
| Template | 2 µL |
| Total | 25.0 µL |

A standard curve is created by plotting the copy number of the RNA Control Template provided with the kit on the X-axis and the Ct value calculated from the second derivative curve (2^{nd} Derivative) on the Y-axis. Table 8 below shows the Ct values of the standard curve for real-time PCR. In the table below, for example, "1.00E+08" represents "1.00 × 10⁸". The same applies herein below.

**[Table 8]**

| STD | Copy number/µL | Ct(SDM) | Ct Ave. |
|---|---|---|---|
| 1 | 1.00E+08 | 15.43 | 15.40 |
| | | 15.37 | |
| 2 | 1.00E+07 | 19.12 | 19.12 |
| | | 19.12 | |
| 3 | 1.00E+06 | 22.72 | 22.78 |
| | | 22.84 | |
| 4 | 1.00E+05 | 26.30 | 26.33 |
| | | 26.35 | |
| 5 | 1.00E+04 | 29.85 | 29.72 |
| | | 29.59 | |
| 6 | 1.00E+03 | 33.19 | 33.12 |
| | | 33.04 | |

The Ct values were calculated by the 2^{nd} 1Derivative Maximum (SDM) method. From the calculated Ct values, we obtained a slope of -3.54, an intercept of 43.88, an amplification efficiency of 91.6, and a coefficient of determination of 0.999. From the standard curve, the calibration curve, the titer of the tested sample was calculated. The results are shown in Table 9.

**[Table 9]**

| Sample name* | Dilution ratio | Ct (SDM) | Calculated copy number (copies/µL) | Titer ** (copies/mL) | Average titer (copies/mL) |
|---|---|---|---|---|---|
| R1601_pDON-5 Neo hTERT | 100 | 26.85 | 6.45E+04 | 1.29E+10 | 1.46E+10 |
| | 100 | 26.89 | 6.28E+04 | 1.26E+10 | |
| | 500 | 28.96 | 1.63E+04 | 1.63E+10 | |
| | 500 | 28.94 | 1.66E+04 | 1.66E+10 | |
| R1601_pDON-5 Neo HPV16E6 | 100 | 27.16 | 5..27E+04 | 1.05E+10 | 1.15E+10 |
| | 100 | 27.15 | 5.30E+04 | 1.06E+10 | |
| | 500 | 29.37 | 1.25E+04 | 1.25E+10 | |
| | 500 | 29.39 | 1.24E+04 | 1.24E+10 | |
| R1601_pDON-5 NeoHPV16E7 | 100 | 25.06 | 2.06E+05 | 4.13E+10 | 4.05E+10 |
| | 100 | 25.14 | 1.96E+05 | 3.92E+10 | |
| | 500 | 27.49 | 4.25E+04 | 4.25E+10 | |
| | 500 | 27.62 | 3.91E+04 | 3.91E+10 | |
| R1601_pDON 5 Neo pigTERT | 100 | 26.94 | 6.08E+04 | 1.22E+10 | 1.27E+10 |
| | 100 | 26.97 | 5.96E+04 | 1.19E+10 | |
| | 500 | 29.26 | 1.35E+04 | 1.35E+10 | |
| | 500 | 29.30 | 1.31E+04 | 1.31E+10 | |
| R1601_pDON-5 Neo hBmi1 | 100 | 25.73 | 1..34E+05 | 2.67E+10 | 2.83E+10 |
| | 100 | 25.82 | 1.26E+05 | 2.52E+10 | |
| | 500 | 27.88 | 3.30E+04 | 3.30E+10 | |
| E.D. | I | 35.92 | 1.77E+02 | - | - |
| | I | 33.94 | 6.41E+02 | - | |

In the above table, E.D. indicates that dilution buffer EAST Dilution (for real-time PCR) was used. The titer (copies/mL) was obtained as calculated copy number (copies/µL) × 2 (dilution ratio for Dnase I treatment) × dilution ratio for Real-Time PCR reaction (100 or 500) × 1,000 (conversion from µL to mL).

Real-time PCR results showed titers of 1.46 × 10¹⁰ copies/mL for DON-5 Neo hTERT, 1.15 × 10¹⁰ copies/mL for pDON-5 Neo HPV16E6, 4.05 × 10¹⁰ copies/mL for pDON-5 Neo HPV16E7, 1.27 × 10¹⁰ copies/mL for pDON-5 Neo pigTERT, or 2.83 × 10¹⁰ copies/mL for pDON-5 Neo hBmi1.

### (Example 2) Production of SHED

Exfoliated dense deciduous teeth from a healthy 10-year-old boy were used. After the teeth are disinfected by using an appropriate disinfection agent such as Isodine, the crown of the tooth is cut in a horizontal direction by using the diamond point for a dentist. Then, the dental pulp tissue is collected both from the dental crown and dental root portions by using a dental reamer. The obtained dental pulp tissue is digested in the solution containing 3 mg/mL of type I collagenase and 4 mg/mL of dispase at 37 °C for 1 hour. Then, the solution is filtrated by using the cell strainer with 70 mm (Falcon).

The filtrated cells are resuspended in 4 mL of the medium and plated in the culture dish for the adherent cell having a diameter of 60 mm. DMEM (Dulbecco's Modified Eagle's Medium) containing 10 % FCS is added into the dish and then incubated for about 2 weeks in the incubator under the presence of 5% CO₂ at 37 °C. The adherent cells that formed the colonies (the dental pulp stem cells) are treated with 0.2 mM EDTA containing 0.05% trypsin for 5 minutes at 37 °C, and then release cells from the dish were collected.

Next, the selected cells described above are plated in the culture dish for the adherent cells (the collagen-coated dish), and then conducted primary culture in the incubator under the presence of 5 % CO₂, at 37 °C to obtain the primary cultured cells. When the cells become subconfluent (about 70 % of the surface was covered by the cells) or confluent by macroscopic observation, they are treated with 0.2 mM EDTA containing 0.05 % of trypsin for 5 minutes, at 37 °C for releasing the cells from the culture dish and then to be collected. Thus, obtained cells are again plated in the dich containing above medium, and passed several times to be grown until the cell number became about 1 × 10⁷ cells/mL. Obtained cells are stored in the liquid nitrogen.

After that, the primary culture cells are cultured by using the above-mentioned medium at a concentration of about 1 × 10⁴ cells/cm². The cells passed 1 to 3 times are used for the experiments. Human BMMSCs (Bone Marrow Mesenchymal stem cells) were purchased from Lonza and cultured according to the manufacturer's instructions. Human deciduous dental pulp stem cells (SHED) are obtained as described above. The resulting SHEDs are sorted using FACSTARPLUS (Becton Dickinson) with approximately 1 × 10⁶ STRO-1 positive cells for each sample as follows.

BrdU is taken up for 12 hours according to the manufacturer's instructions for the bromodeoxyuridine BrdU staining kit (Invitrogen) and the growth rate of SHED is evaluated (n=3 for each group). The experiment was repeated five times; after one-way analysis of variance, the Tukey-Kramer test was performed to evaluate statistically significant differences.

To detect STRO-1 by immunofluorescence, SHEDs are fixed with 3% paraformaldehyde, then rinsed twice with PBS, and treated with 100 mM glycine for 20 minutes. These cells are then permeabilized with 0.2% Triton-X (Sigma-Aldrich) for 30 minutes, followed by incubation in a mixture of 5% donkey serum and 0.5% bovine serum albumin for 20 minutes.

Next, the cells are incubated for 1 hour with the primary antibody, mouse antihuman STRO-1 antibody (1:100, R&D), incubated for 30 minutes with the secondary antibody, goat anti-mouse immunoglobulin M-FITC antibody (1:500, Southern Biotech), and then mounted with Vectashield DAPI (Vector Laboratories Inc.). Then, α-MEM supplemented with 15% FBS is placed in a 6-well plate and the sorted cells are spread for cloning. Approximately 300 colonies from the proliferated cells are pooled for testing.

### (2) Gene introduction

### (2-1) Gene introduction with the Adenovirus vector

As described above, four genes, bmi-1, E6, E7 and hTERT, were incorporated into adenovirus vectors to create viral vectors expressing these gene products. As a control, a control vector was created that did not incorporate these genes.

SHED is spread on a 100 mm diameter collagen-coated dish at 1 × 10⁶ cells and incubated with DMEM supplemented with 10% FBS until subconfluent. Then, the medium is aspirated off, and 500 µL of virus solution diluted in the medium is added to the dish (MOI=10) and incubated at 37 °C for 1 hour in a 5 % CO₂ incubator to infect with the above virus vector. After 48 hours of infection, infected cells are cultured for 10 days in the medium added with puromycin (1 pg/mL) for selection, and 500-600 resistant clones are pooled. Every 3-4 days, about 0.5 × 10⁵ SHEDs are inoculated into 100 mm diameter culture petri dishes for passage. SHEDs with transgenes are designated SHED-T and SHEDs without transgenes were designated SHED-C.

### (2-2) Gene introduction with the retrovirus vector

The retrovirus vectors obtained as described above are infected to human dental pulp-derived cells (KA_01_W14_P5D3) or human dental pulp-derived cells (KA_02_W4-6_P4D3) to obtain drug-resistant pool cells (hereinafter simply referred to as "pool cells"). The obtained pool cells are subjected to real-time PCR for analysis to confirm the number of inserted genes. Primers listed in Table 10 below (all from Takara Bio) were used for real-time PCR.

**[Table 10]**

| Gene | Catalogue No. | Base sequence | SEQ ID NO.. |
|---|---|---|---|
| Target gene | CH000987_F | GCACTGCCCTCAGACTTCAAGA | 16 |
| | CH000987_R | GCGGGACTATGGTTGCTGAC | 17 |
| Standard gene (Human β actin) | HA067803_F | TGGCACCCAGCACAATGAA | 18 |
| | HA067803_R | CTAAGTCATAGTCCGCCTAGAAGCCA | 19 |

The KA_01_W14_P5103 cells in a Corning adherent cell culture dish (6 or 10 cm diameter Cell culture Dish TC treated surface) are cultured at 37 °C in a 5 % CO₂ incubator. The number of passages when these cells are raised and seeded was denoted as [0] (hereafter "+P0"), "+1" for one passage, "+2" for two passages, and so on.

When the cells in the dish reach about 80 % confluence, the culture supernatant is removed. Then, the adherent cells are washed 2-3 times with PBS; detached from the bottom of the dish by treating them with 0.25 % trypsin-EDTA (1x) (containing phenol red) (Thermo Fisher Scientific, 25200-056) at 37 °C in a 5 % CO₂ incubator for 3 minutes.

An appropriate amount of medium is added to the cells and pipetted to prepare a single-cell suspension. The cell suspension is diluted to an appropriate volume and transferred to a new dish. The culture is continued in the same manner, and it is confirmed that there were no abnormalities in cell proliferation or morphology. The culture supernatant collected at the time of passaging is also checked for mycoplasma contamination using the MycoAlert Mycoplasma Detection Kit (Loza, product code LT07-318).

### (3) Examination of drug resistance concentrations

To select gene-introduced cells by drug, G418-resistant concentrations of target cells are examined. 2.0 × 10⁴ cells in the logarithmic growth phase are inoculated in each well of Corning 6-well plates (6 Well Clear TC-Treated Multiple Well Plate, product code 3516) and incubated at 37 °C in a 5 % CO₂ incubator for approximately 24 hours, after which cell adhesion to the bottom of the wells was checked. The medium in each well is replaced with medium supplemented with different concentrations of G418. The condition of the cells was imaged 4, 8, and 10 days after the start of drug selection to examine the drug resistance concentration.

4 days after the start of drug selection, cell growth activity is lost in wells with a drug concentration of 250 µL/mL or higher, and 11 days later, most cells in the wells with a drug concentration of 500 µL/mL or higher were found to be dead. Therefore, the G418 selective drug concentration for target cells is determined to be 500 µL/mL, which shows sufficient selective effect.

The target cells are infected with retrovirus vector solution by the polybrene method, and immortalized gene stable expression lines were selected by medium B. 1.40 × 10⁵ cells in the logarithmic growth phase are seeded in each well of the 6-well plate and incubated at 37 °C in a 5 % CO₂ incubator for about 24 hours. Then, recombinant retrovirus vector solution diluted 4-fold or 10-fold in medium A is added to a final concentration of 8 µg/mL of polybrene (Hexadimethrine bromide, Sigma-Aldrich, product code H9268), and the same amount is added to each well to infect the cells with the virus. Approximately 4 hours after virus infection, 1.0 mL of medium A is added to each well. The plate is incubated at 37 °C in a 5 % CO₂ incubator and replaced with medium B after 24 hours to initiate drug selection.

After 10 days from the start of the drug selection, cells that are drug-resistant and proliferated are collected. 1 mL of CELLBANKER 1 Plus (Nippon Zenyaku Kogyo, product code CB021) is added to the cell to make a suspension containing 1 × 10⁶ cells/mL, which was stored at -80 °C as cell stock to be received. At the same time, a cell pellet of 5 × 10⁵ cells/vial was prepared for real-time PCR analysis and stored at -80 °C.

### (Example 3) Analysis of factors in the conditioned medium

### (1) Preparation of the conditioned medium of the immortalized stem cells

2 × 10⁵ cells/mL of the immortalized stem cells obtained in Example 1 and 2 (herein below referred to as "hSHED") are placed in a culture dish (55 cm²) containing 10 mL of Dulbecco's modified Eagle's medium (herein below abbreviated as "DMEM") supplemented with 10% fetal bovine serum (herein below referred to as "FBS") and incubated at 37 °C in a 5 % CO₂ incubator until 70 % confluent (4 to 6 days).

After confirming 70 % confluent with microscopy, the culture supernatant is removed by aspiration, and the cells are washed with serum-free DMEM (herein below sometimes referred to as "Plain DMEM") twice. Subsequently, 10 mL of serum-free DMEM is added to the culture flasks. The culture supernatant collected immediately after the addition is used as a control. After the addition, the cells are incubated at 37 °C for 72 hours in a 5 % CO₂ incubator. The culture supernatant is collected in a 15 mL sterilized centrifuge tube, centrifuged at 1,500 rpm (approximately 440 × g) for 3 minutes, and the centrifuged supernatant is passed through a 0.22 mm PVDF filter (Millipore) to obtain the conditioned medium of immortalized hSHED (herein below sometimes referred to as "hSHED-CM").

### (2) Quantitative expression analysis

According to the instructions accompanying the kit, namely following procedures, the quantitative expression analysis is performed to identify what kind of cytokines are expressed in the obtained hSHED-CM using Quantibody (Registered trademark) Human Cytokine Antibody Array 1000 (RayBiotech Inc., catalog No. QAH-CAA-1000) (Figure 3).

First, the SHED-CM is contacted with immobilized antibodies in array arrangement on a support for 1 to 2 hours. Then, a cocktail of biotinylated antibodies is added to each well and incubated for 1 to 2 hours. Subsequently, labeled streptavidin is added thereto and incubated for 2 hours to detect the labeling signal. Data analysis thereof is performed using GenePix (Registered trademark) 4400A (Molecular Dveices, LLC). Results are shown in Figure 1. 80 cytokines were measured, and 36 cytokines among them having a difference of 1 pg/mL or more from the control are shown in ascending order of concentration. Table 11 below shows the names of the 80 cytokines measured.

**[Table 11]**

| No. | Cytokine name | No. | Cytokine name | No. | Cytokine name |
|---|---|---|---|---|---|
| 1 | BLC/BCA-1/CXCL13 | 30 | MIP-1α/CCL3 | 58 | IGFBP-1 |
| 2 | Eotaxin/CCL11 | 31 | MIP-1β/CCL4 | 59 | IGFBP-2 |
| 3 | Eotaxin-2/CCL24 | 32 | MIP-16/CCL15 | 60 | IGFBP-3 |
| 4 | G-CSF | 33 | PDGF-BB | 61 | IGFBP-4 |
| 5 | GM-CSF | 34 | RANTES/CCL5 | 62 | IGFBP-6 |
| 6 | I-309/CCL1 | 35 | TIMP-1 | 63 | IGF-I |
| 7 | ICAM-1 | 36 | TIMP-2 | 64 | Insulin(pro & mature) |
| 8 | IFNγ | 37 | TNFα | 65 | M-CSF R |
| 9 | IL-1α | 38 | TNFβ | 66 | NGF R |
| 10 | IL-1β | 39 | sTNFR I/TNFRS1A | 67 | NT-3 |
| 11 | IL-1ra | 40 | sTNFR II/TNFRS1B | 68 | NT-4 |
| 12 | IL-2 | 41 | Amphiregulin(AR) | 69 | Osteoprotegerin(OPG) |
| 13 | IL-4 | 42 | BDNF | 70 | PDGF-AA |
| 14 | IL-5 | 43 | bFGF/FGF-2 | 71 | PIGF |
| 15 | IL-6 | 44 | BMP-4 | 72 | SCF |
| 16 | IL-6 sR | 45 | BMP-5 | 73 | SCF R |
| 17 | IL-7 | 46 | BMP-7 | 74 | TGFα |
| 18 | IL-8/CXL8 | 47 | β-NGF | 75 | TGFβ1 |
| 19 | IL-10 | 48 | EGF | 76 | TGFβ3 |
| 20 | IL-11 | 49 | EGF R | 77 | VEGF |
| 21 | IL-12p40 | 50 | EG-VEGF | 78 | VEGF R2 |
| 22 | IL-12p70 | 51 | FGF-4 | 79 | VEGF R3 |
| 23 | IL-13 | 52 | FGF-7/KGF | 80 | VEGF-D |
| 24 | IL-15 | 53 | GDF-15 | | |
| 25 | IL-16 | 54 | GDNF | | |
| 26 | IL-17 | 55 | Growth Hormone | | |
| 27 | MCP-1/CCL2 | 56 | HB-EGF | | |
| 28 | M-CSF | 57 | HGF | | |
| 29 | MIG/CXCL9 | | | | |

Figure 1 showed that the content amounts of the insulin-like growth factor binding proteins, IGFBP-4, IGFBP-6, and IGFBP-2, and the metalloproteinase tissue inhibitors, TIMP-1 and TIMP-2, were relatively high. Insulin was omitted, because it was not measured correctly in this study.

### (3) Qualitative expression analysis

According to the instructions accompanying the following kit, the qualitative expression analysis is performed to identify what kind of cytokines are expressed in the obtained hSHED-CM using RayBio (Registered trademark) Human Cytokine Antibody Array G-Series 4000 (RayBiotech Inc., catalog No. AAH-CYT-G4000-4) (Figure 4).

First, the SHED-CM or standard protein cocktail is contacted with an array of antibodies immobilized on glass slides for 1 to 2 hours, followed by incubation with a cocktail of biotinylated antibodies for 1 to 2 hours. Subsequently, the samples are incubated with Cy3 equivalents of labeled streptavidin for 1 hour, followed by scanning, data extraction, and data analysis using GenePix (registered trademark) 4400A (Molecular Dveices, LLC).

By this qualitative analysis, 77 of which had an expression intensity of 1.5 or greater relative to controls among the measured 264 cytokines (Tables 12-16 below). Figures 7 and 8 show the 77 cytokines listed above with relative expression intensities of 3 or greater.

**[Table 12]**

| Cytokine name | | | | | |
|---|---|---|---|---|---|
| Angiogenin | BDNF | BLC | BMP-4 | BMP-6 | CKb8-1 |
| CNTF | EGF | Eotaxin | Eotaxin-2 | Eotaxin-3 | FGF-6 |
| FGF-7 | FLT-3 Ligand | Fractalkine | GCP-2 | GDNF | GM-CSF |
| 1-309 | INF-Gamma | IGFBP-1 | IGFBP-2 | IGFBP-4 | IGF-1 |
| IL-10 | IL-13 | IL-15 | IL-16 | IL-1alpha | IL-1beta |
| IL-1ra | IL-2 | IL-3 | IL-4 | IL-5 | IL-6 |
| IL-7 | Leptin | LIGHT | MCP-1 | MCP-2 | MCP-3 |
| MCP-4 | M-CSF | MDC | MIG | MIP-1 delta | MIP-3 alpha |
| NAP-2 | NT-3 | PARC | PDGF-BB | RANTES | SCF |
| SDF-1 | TARC | TGF-beta1 | TGF-beta3 | TNF-alpha | TNF-beta |

**[Table 13]**

| Cytokine name | | | | | |
|---|---|---|---|---|---|
| Acrp30 | AgRP | Angiopoietin-2 | Amphiregulin | Axl | bFGF |
| b-NGF | BTC | CCL-28 | CTACK | Dtk | EGF-R |
| ENA-78 | Fas/TNFRSF6 | FGF-4 | FGF-9 | GCSF | GITR-Ligand |
| GITR | GRO | GRO-alpha | HCC-4 | HGF | ICAM-1 |
| ICAM-3 | IGFBP-3 | IGFBP-6 | IGF-I SR | IL-1 R4/ST2 | IL-1 RI |
| IL-11 | IL-12 p40 | IL-12 p70 | IL-17 | IL-2 Rapha | IL-6 R |
| IL-8 | I-TAC | Lymphotactin | MIF | MIP-1 alpha | MIP-1 beta |
| MIP-3 beta | MSP-alpha | NT-4 | Osteo -protegerin | Oncostatin M | PIGF |
| sgp130 | sTNF RII | sTNF-RI | TECK | TIMP-1 | TIMP-2 |
| Thrombo -poietin | TRAIL R3 | TRAIL R4 | uPAR | VEGF | VEGF-D |

**[Table 14]**

| Cytokine name | | | | | |
|---|---|---|---|---|---|
| Activin A | ALCAM | B7-1(CD80) | BMP-5 | BMP-7 | Cardiotrophin-1 |
| CD14 | CXCL-16 | DR6 (TNFRSF21) | Endoglin | ErbB3 | E-Selectin |
| FAS Ligand | ICAM-2 | IGF-II | IL-1RII | IL-10 Rbeta | IL-13 Ralpha2 |
| IL-18 BPalpha | IL-18 Rbeta | IL-2 Ralpha | IL-2 Rbeta | IL-2 Rgamma | IL-21R |
| IL-5 Ralpha | IL-9 | IP-10 | LAP | Leptin R | LIF |
| L-Selectin | M-CSF R | MMP-1 | MMP-13 | MMP-9 | MPIF-1 |
| NGF R | PDGF AA | PDGF-AB | PDGF Ralpha | PDGF Rbeta | PECAM-1 |
| Prolactin | SCF R | SDF -1 beta | Siglec-5 | TGF-alpha | TGF beta2 |
| Tie-1 | Tie-2 | TIMP-4 | VE-Cadherin | VEGF R2 | VEGF R3 |

**Table 15]**

| Cytokine name | | | | |
|---|---|---|---|---|
| Adipsin | BCAM | CD30 | CD40 | Fcgamma RIIB/C |
| Ferritin | FLRG/FSTL3 | Follistatin | Furin | Galectin-7/ LGALS7 |
| GDF-15/MIC-1 | Growth Hormone(GH) | IL-10 R alpha | IL-22 | IL-28A/ IFN-gamma2 |
| IL-29 | IL-31 | Insulin (pro & mature) | LH | LIMPII/SR-B2 |
| LYVE-1 | Marapsin/ Pancreasin | MICA | MICB | MMP-2 |
| MMP-7 | MMP-8 | MMP-10 | NCAM-1/CD56 | Nidogen-1/ Entactin |
| NrCAM/Bravo | NRG1 beta 1 | Osteopontin (OPN) | PAI-1 | PF4 |
| PSA(total) | RAGE/AGER | RANK/ TNFRSF11A | Resistin | Serum Amyloid A (SAA) |
| Siglec-9 | TACE/ CD156b | TIM-1/HAVCR | TRAIL R3/ TNFRSF10B | Trappin-2 |
| TREM-1 | TSH | TSLP | VCAM-1 | VEGF-C |
| XEDAR | - | - | - | |

**[Table 16]**

| Cytokine name | | | | |
|---|---|---|---|---|
| 4-1BB | ACE-2 | Alpha-fetoprotein | Angiopoietin-1 | Angiostatin (ANG) |
| ANGPTL4 | Beta-2 Micro-globulin | BCMA/TNFRSF17 | beta-IG-H3/ TGFBI | CA 125 |
| CA15-3 | CA19-9 | Carbonic Anhydrase IX | Cathepsin S | CCL14a/ HCC-1 |
| CCL21/ 6Ckine | CD23/Fc epsilon-RII | CD40 Ligand | CEA/CD66e | CEACAM-1 |
| Cripto-1/CR1 | CRP | DAN/NBL1 | Decorin (DCN) | Dickkopf-1 (Dkk-1) |
| Dickkopf-3 (Dkk-3) | Dickkopf-4 (Dkk-4) | DPPIV/CD26 | E-Cadherin | EDA-A2 |
| EG-VEGF | Ep-CAM/ CD326 | ErbB2/HER2 | Erythropoeitin R (Epo R) | FSH |
| HB-EGF | hCGa (intact) | HVEM/TNFRSF 14 | IL-13 R alpha-1 | IL-17B |
| IL-17C | IL-17F | IL-17R | Procalcitonin (PCT) | PSA (free) |
| S100b | Shh N | Thyroglobulin (TG) | Ubiquitin+1 | - |

As a result, the relative expression intensities of NGF, IL family (IL-6, IL-8, IL6R, IL-17C, IL-17R, etc.), and others were less than 10.

### (Example 4) Expression analysis of microRNAs in exosomes

Expression analysis of microRNAs in exosomes which are membrane vesicles derived from endosome are performed according to the following procedures.

First, the immortalized stem cells established in Example 2 are cultured in a serum-free medium as described in Example 3, and approximately 10 mL of the conditioned medium is collected. Exosomes were purified from the conditioned medium using ExoQuicl-TC (System Biosciences), according to the manual provided with this kit.

Both miRNA and total RNA are extracted from purified exosomes using the miRNeasy Mini Kit (manufactured by Quiagen), labeled as follows, and then analyzed for cytokine expression by using the GeneChip (registered trademark) miRNA Array (Affymetrix) (Figure 5).

RNA molecules of miRNA and total RNA extracted from the purified exosomes are reacted with polyA polymerase in the presence of ATP for 15 minutes to add polyA tail to make polyA-tailed mRNA. Then, the biotin-labeled RNA is reacted with ligase in Flash Tag Ligation Mix for 30 minutes to ligate the biotin-labeled RNA and the mRNA with polyA tail to obtain labeled molecules as shown in Figure 6. Here, hSHED-CM obtained by culturing the immortalized stem cells for 72 hours is used as the sample.

The labeled molecules are subjected to GeneChip-based assays and detected with fluorescent-labeled streptavidin. The detected results are shown in Table 17 from Nos. 1 to 100 in descending order of expression. Among 6,599 of total microRNAs analyzed by the GeneChip miRNA Array, 845 were positive.

**[Table 17]**

| No. | Abbreviation | No. | Abbreviation | No. | Abbreviation |
|---|---|---|---|---|---|
| 1 | hsa-miR-6126 | 35 | hsa-miR-1237-5p | 69 | hsa-miR-6816-5p |
| 2 | hsa-miR-3960 | 36 | hsa-miR-6125 | 70 | hsa-miR-125b-5p |
| 3 | hsa-miR-24-3p | 37 | hsa-miR-6780b-5p | 71 | hsa-mir-6800 |
| 4 | hsa-miR-3613-3p | 38 | hsa-miR-3178 | 72 | hsa-miR-619-5p |
| 5 | hsa-miR-6087 | 39 | hsa-miR-320b | 73 | hsa-miR-3940-5p |
| 6 | hsa-miR-7704 | 40 | hsa-miR-3656 | 74 | hsa-miR-6805-5p |
| 7 | hsa-miR-4787-5p | 41 | hsa-miR-8069 | 75 | hsa-miR-7108-5p |
| 8 | hsa-miR-1273g-3p | 42 | hsa-miR-320c | 76 | hsa-miR-7150 |
| 9 | hsa-miR-4668-5p | 43 | hsa-miR-100-5p | 77 | hsa-miR-328-5p |
| 10 | hsa-miR-23a-3p | 44 | HBII-85-26 | 78 | hsa-miR-222-3p |
| 11 | hsa-miR-4484 | 45 | hsa-miR-221-3p | 79 | hsa-miR-4454 |
| 12 | hsa-miR-4497 | 46 | hsa-miR-638 | 80 | hsa-miR-320d |
| 13 | hsa-miR-3665 | 47 | hsa-miR-3135b | 81 | hsa-mir-6722 |
| 14 | hsa-miR-1246 | 48 | hsa-let-7b-5p | 82 | hsa-miR-663a |
| 15 | hsa-miR-149-3p | 49 | hsa-miR-6724-5p | 83 | hsa-miR-4487 |
| 16 | hsa-miR-6869-5p | 50 | hsa-miR-6786-5p | 84 | hsa-miR-191-5p |
| 17 | hsa-miR-2115-5p | 51 | hsa-miR-762 | 85 | hsa-miR-6743-5p |
| 18 | hsa-miR-6089 | 52 | hsa-mir-320e | 86 | hsa-let-7c-5p |
| 19 | hsa-miR-8075 | 53 | hsa-miR-214-3p | 87 | hsa-miR-4281 |
| 20 | hsa-miR-6090 | 54 | hsa-miR-1228-5p | 88 | hsa-miR-6800-5p |
| 21 | hsa-miR-1908-5p | 55 | hsa-miR-6732-5p | 89 | ENSG00000238388 |
| 22 | hsa-miR-6729-5p | 56 | hsa-miR-4687-3p | 90 | hsa-miR-6765-5p |
| 23 | hsa-miR-8072 | 57 | hsa-miR-4530 | 91 | hsa-miR-4745-5p |
| 24 | hsa-miR-3196 | 58 | hsa-miR-6803-5p | 92 | hsa-miR-27a-3p |
| 25 | hsa-miR-6088 | 59 | hsa-miR-23b-3p | 93 | hsa-miR-4749-5p |
| 26 | hsa-miR-4488 | 60 | hsa-miR-1469 | 94 | ACA57 |
| 27 | hsa-miR-1915-3p | 61 | hsa-mir-711 | 95 | hsa-miR-134-5p |
| 28 | hsa-miR-4508 | 62 | HBII-85-6 | 96 | hsa-miR-4467 |
| 29 | hsa-miR-145-5p | 63 | hsa-miR-92a-3p | 97 | hsa-miR-6775-5p |
| 30 | hsa-miR-4516 | 64 | hsa-miR-2861 | 98 | ENSG00000238388 |
| 31 | hsa-miR-320a | 65 | hsa-mir-320a | 99 | hsa-miR-6085 |
| 32 | hsa-miR-6727-5p | 66 | hsa-miR-26a-5p | 100 | hsa-miR-1207-5p |
| 33 | hsa-miR-4466 | 67 | hsa-miR-16-5p | | |
| 34 | hsa-miR-5787 | 68 | hsa-miR-130b-3p | | |

### (Example 5) Examination of pressure ulcer treatment effect

### (1) Production of a model animal

By utilizing the ischemia/reperfusion method, pressure ulcers, one of the ischemia-reperfusion disorders, are induced in mice. hSHED-CM is administered to the mice, and the process of pressure ulcer healing is observed. Here, the ischemia-reperfusion injury refers to various disorders that are caused in tissues that have become ischemic (hypoxic) due to the interruption of blood flow (oxygen) supply, when blood flow is reperfused thereto.

Pressure ulcer model mice are created by sandwiching the dorsum skin of 8 weeks old C57/BL6 (female) mice with 1180-gauss magnets for 12 hours to generate the sites with ischemic states (ischemic sites) (see Figure 9). Here, Figure 9 shows that the sites sandwiched by the magnets were in a strong ischemic state, that by this pressure ulcers and other lesions were caused when damage occurred within the site, and that weak ischemia already occurred around the strong ischemic sites due to a reduction in blood flow, resulting in hair loss. The administration schedule for this model mouse, and the time-dependent changes in the pressure ulcer sites after the magnets are removed are shown in below.

### (2) Administration schedule and progress

### (2-1) Administration schedule 1 (4-day consecutive dosing from after the day of I/R treatment, one day interval, and then 2-day consecutive dosing)

The pressure ulcer model mice (negative control group: 5 mice, positive control group: 5 mice, and test group: 5 mice) prepared in (1) are used. For all groups, the start of ischemia is set as Day 0, and from Day 1 to Day 4, DMEM is subcutaneously administered to the negative control group, and 1 µg/100 µL of bFGF is subcutaneously administered to the positive control group, on their dorsum. 100 µL of hSHED-CM (equivalent to 250 pg of HGF) is subcutaneously administrated to the dorsum of the test group every day. Thereafter, on Day 5, no doses were administered to all groups, and the same doses were administered subcutaneously to the dorsum of the model mice on Day 6 and Day 7 (see Figures 10(A) and 10(B)).

The number of days after reperfusion and the percentage of wound area (Pressure ulcer-formed area) are shown in Figure 10(C). Compared to the control, both groups treated with hSHED-CM or bFGF showed a rapid reduction in the percentage of the wound area.

### (2-2) Administration schedule 2 (7-day consecutive dosing after the day of I/R treatment - 1)

The pressure ulcer model mice (negative control group: 5 mice, test group: 5 mice) prepared in (1) above are used. DMEM or hSHED-CM is administered to the pressure ulcer formation sites (2 sites) of the model mice in 100 µL each to the control or test groups, respectively, as described in (2-1) above (see Fig. 11(A) and (B)). The results are shown in Figure 11(C). hSHED-CM-treated group showed a significantly smaller tendency to enlarge the percentage of the wound area immediately after reperfusion compared to these of the control group, and also showed a rapid and significant reduction in the size of the wound area until day 10 (see Figure 11(C)). In addition, comparison of the conditions of the skin where the pressure ulcer was formed showed that the skin of the test group is more quickly recovered than that of the control group, and that the pressure ulcer in the test group had almost completely cured by Day 12 (see Figure 12).

### (2-3) Administration schedule 3 (7-day consecutive dosing from the next day of the I/R treatment)

The pressure ulcer model mice (the negative control group: 5 mice, the test group: 5 mice) prepared in (1) are used. DMEM or hSHED-CM is administered to the pressure ulcer formation sites (2 sites) of the model mice in 100 µL each to the control group or test group, respectively, as described in (2-2) (Fig. 13(A)-(C)). Then, on Day 4, the skins of the wound area are collected, fixed in formalin, and immunologically stained for 8-OHdG, CD31, or NG2 (Fig. 14(A)-(C)). The results of each staining are shown in Figure 15(A)-(B), Figure 16(A)-(B) and Figure 17(A)-(B).

### (2-4) Administration schedule 4 (7-day consecutive dosing after the pressure ulcer development)

In order to confirm curing after pressure ulcer development, no medical treatment is given to the groups for 5 days after I/R treatment, and then 7-day consecutive dosing was performed to both groups from 6 days after I/R treatment after the formation of the pressure ulcer.

The pressure ulcer model mice (the negative control group: 5 mice, the test group: 5 mice) prepared in (1) are used. DMEM or hSHED-CM is administered to the pressure ulcer formation sites (2 sites) of the model mice in the same manner as described above (2-1), 50 µL each, to the control or test group, respectively (see Fig. 18(A) and (B)). The results are shown in Figure 18(C).

Compared to the control group, the hSHED-CM-administrated group showed a significant reduction in the size of the wound formation area after hSHED-CM administration. In addition, comparison of the conditions of the skin where the pressure ulcer was formed showed that the skin of the test group was more quickly recovered than that of the control group, and that the pressure ulcer in the test group had almost completely cured by Day 10 (see Figure 19).

### (2-5) Administration schedule 5 (7-day consecutive dosing of the conditioned medium without a target factor, immediately after the I/R treatment)

In order to examine the curing effect of the conditioned medium of h-SHED or the primary cells from which cytokines are removed, cytokines included thereof are analyzed as described in Example 3. Human-derived Primary dental pulp stem cell purchased from Cell Technology, Inc. is used as the primary cells. The results are shown in Table 13. Of these, since the content amounts of IGFBP-4, VEGF, and HGF and tissue repair/regeneration capacity thereof are high , they are set as target factors in the immunoprecipitation described below.

### (2-6) Administration schedule 6 (7-day consecutive dosing from 7 day before the I/R treatment)

In order to examine whether pre-administration of the culture supernatant is effective in prevention of the pressure ulcer development, hSHED-CM is administered for 7 consecutive days before I/R treatment. Pressure ulcer model mice (the negative control group: 4 mice, the test group: 4 mice) prepared as described in (1) are used. 100 µL of DMEM or hSHED-CM is administered to the pressure ulcer formation sites (2 sites) of the model mice in the same manner as described in (2-1), in the control group or the test group, respectively (see Figure 26(A) and (B)). The results are shown in Figures 27 and 28.

Table 18 below summarizes the amounts of various factors, cytokines, and the like, in the conditioned medium, when the immortalized stem cells of the present invention or the primary cells are cultured. In Table 18, the primary cultured cells are indicated as "primary".

**[Table 18]**

| No. | Cytokine name (abbreviation and name) | | Concentration in conditioned medium (pg/mL) | | | Function |
|---|---|---|---|---|---|---|
| | | | Immortalized | Primary | | |
| 1 | IGFBP-4 | Insulin-like growth factor binding protein | 457,262 | 18,592 | Inhibition of tumor growth and angiogenesis | |
| 2 | IGFBP-6 | | 27,919 | 3,907 | | |
| 3 | IGFBP-2 | | 27,908 | 72 | | |
| 4 | VEGF vascular endothelial growth factor | | 2,734 | 90 | Angiogenesis, hair growth, hair restore | |
| 5 | HGF hepatocyte growth factor | | 2,500 | 1,521 | | |
| 6 | MCP-1 chemokine/monocyte migration factor | | 1,347 | 851 | | |
| 7 | TNF RII tumor necrosis factor receptor | | 1,233 | 6 | | |
| 8 | IGFBP-3 Insulin-like growth factor binding protein | | 1,166 | 98 | | |
| 9 | RANTES chemokine | | 374 | 36 | | |
| 10 | AR androgen receptor | | 303 | 54 | | |
| 11 | TNFb tumor necrosis factor | | 279 | 0 | | |
| 12 | EGF R epidermal growth factor receptor | | 264 | 56 | | |
| 13 | IL-6 inflammatory cytokine | | 193 | 6 | | |
| 14 | EGF-7 keratinocyte growth factor | | 151 | 45 | hair growth, hair restore | |
| 15 | IL-8 inflammatory cytokine/ chemokine | | 104 | 11 | | |
| 16 | Eotaxin chemokine/acidophilic leukocyte migration factor | | 88 | 1 | | |
| 17 | PIGF placental growth factor | | 67 | 4 | | |
| 18 | GDF-15 growth differentiation factor 15 | | 34 | 39 | | |
| 19 | SCF R stem cell factor receptor | | 29 | 10 | | |
| 20 | MCSF macrophage colony stimulation factor | | 22 | 0 | | |

Conditioned medium of immortalized stem cells cultured under the conditions described in Example 3 is collected in the sterilized tubes and immunoprecipitated with Protein G Sepharose (Cytiva) and antibodies (α-IGFBP-4, α-VEGF, and α-HGF, R&D) to prepare a conditioned medium with the target factors removed.

The conditioned medium obtained as described above is administered to the pressure ulcer formation sites (two sites) of the model mice prepared in (1), 100 µL each, two mice in each group. DMEM is administered to the negative control group, and hSHED-CM to the positive control group. Here, hSHED-CM is used, in which, based on the results of component analysis of the culture supernatant, any one of IGFBP-4, VFGF (vascular endothelial growth factor), and HGF (hepatocyte growth factor) is depleted by immunoprecipitation technique using specific antibodies against each of these molecules. In Figure 20(A), "IP" is an abbreviation for "immunoprecipitation". The administration schedule was the consecutive dosing from Day 1 to Day 7, with Day 0 as the day on which the mice underwent I/R treatment (see Figure 20(A)). The administration of 100 µL each is conducted at two sites on the back of the I/R-treated mice, as shown in Figure 20(B). The factors administered and the number of animals used (N) are shown in Figure 20(B).

The administration results are shown in Figures 21(A)-21(D). Figure 21(A) shows the result of negative control, wherein the culture supernatant of the present invention treated with a control antibody was used (represented as "control Ab" in Figure 20(B) and Figure 21(A)). Figure 21(B) shows the result of the IGFBP-4 depletion using an anti-IGFBP-4 antibody (represented as "α-IGFBP-4" in Figure 20(B) and Figure 21(B)), Figure 21(C) shows the result of the VEGF depletion using an anti-VFGF antibody (represented as "α- VEGF" in Figure 20(B) and Figure 21(C)). Figure 21(D) shows the result of HGF depletion using an anti-HGF antibody (represented as "α-HGF" in Figure 20(B) and Figure 21(D)). In the figures, * indicates P<0.05, ** indicates P<0.01, and *** indicates P<0.001.

There was no significant difference, when hSHED-CM treated with α-IGFBP-4 (IGFBP-4 depleted hSHED-CM) was administered compared to untreated hSHED-CM. In contrast, there was a significant difference between hSHED-CM treated with α-HGF (HGF-depleted hSHED-CM) and untreated hSHED-CM, with a delayed expansion of the wound area and delayed curing compared to untreated hSHED-CM. When hSHED-CM treated with α-VEGF (VEGF-depleted hSHED-CM) was administered, the wound area expanded broader and cured slower than when untreated hSHED-CM was administered. Thus, we conclude that HGF and VEGF contribute to the healing of pressure ulcers.

### (2) Examination of inhibitory effect of reactive oxygen using the conditioned medium of the cells

It has been reported in the following literature (Protective effect of mesenchymal stem cells on the pressure ulcer formation by the regulation of oxidative and endoplasmic reticulum stress) that mesenchymal stem cells (herein below it is abbreviated as "MSCs") themselves are involved in the curing of pressure ulcers by suppressing reactive oxygen species (herein below it is referred to as "ROS"). Therefore, in vitro studies were conducted to verify whether the conditioned medium of MSC had a similar effect instead of MSC themselves (see Fig. 22).

Figure 22 shows the experimental process to reproduce the pathogenesis of the pressure ulcer model in an in vitro system. As shown in Fig. 23, NIH3T3 is cultured with the conditioned medium of the present invention, in which HGF or VEGF is depleted by immunoprecipitation with specific antibodies to confirm ROS suppression by each cytokine. As the positive control, t-butyl hydroxy peroxidase (herein below, it is sometimes referred to as "TBHP".) is used.

The experiment is performed using NIH3T3 cells according to the following procedures (see Figure 22). NIH3T3 cells are inoculated at 3.0 × 10⁴ cells/well into 8-well slide chambers with clear inner bottom surfaces and cultured overnight in DMEM medium at 37 °C in a 5 % CO₂ incubator. Cell adhesion in each well is checked under the microscope, and the slide chamber is washed with 1 × PBS. DMEM medium supplemented with 1.0 % FBS pre-exposed to 1.0 % oxygen for 72 hours is added to the slide chamber and cultured for 36-48 hours in a 5 % CO₂ incubator at 37 °C under 1.0% oxygen. The cells are then incubated in a fermenter under normal 20.0 % oxygen for 5-6 hours at 37 °C in a 5 % CO₂ incubator.

The cells are then washed again with 1 × buffer provided with 2',7'-dichlorofluorescein diacetate (herein below it is referred to as "DCFDA") Cellular ROS Detection Assay kit (Abeam). Then, 200 µL of 10-20 µM DCFDA dissolved in 1 × buffer is added to each well, and the cells are incubated at 37 °C for 45 minutes to incorporate DCFDA. The ROS generated in the cells are detected as fluorescence of DCF (dichlorofluorescein) using a fluorescence microscope at Ex/Em (excitation wavelength/fluorescence wavelength) = 480/527 nm.

When DCFDA taken up by cells is oxidized by ROS, it turns DCF that emits fluorescence with high intensity. Using the property that its intensity is proportional to the intracellular ROS level; the generation amount of ROS is measured. Specifically, DCF, which is increased in proportion to the generation amount of ROS, is irradiated with excitation light having 480 nm to be quantified from the intensity of fluorescence . As the control, 155 nM TBHP is used, and ROS is quantified from the contrast between the dark and bright areas of the obtained observation. The results are shown in Figures 23 and 24. The contrast calculated from the maximum and minimum brightness of the image shown in Figure 23(A) was used as the relative value of ROS, quantitatively compared and shown in Figure 24.

NIH3T3 cultured typically is used as a negative control group, and NIH3T3 cultured in TBHP-supplemented medium is used as a positive control group. In both controls, the oxygen concentration during cultivation is 20.0 % as usual. In the negative control group, the contrastive value of the signal indicating the occurrence of ROS was approximately 50. In contrast, the positive control group showed about three times stronger values. In addition, a stronger ROS signal, about double than that of the negative control group, was detected in the cells cultured under 1.0% oxygen for 36 to 48 hours and then under 20.0 % oxygen (such culture is hereinafter referred to as "reoxygenation"). In contrast, the ROS signal from the cells cultured in the same manner as above supplemented with the conditioned medium of the present invention showed the same level as that of the negative control group. However, when HGF and VEGF were depleted from the conditioned medium, the ROS signal increased. These results indicate that hSHED-CM has ROS production inhibitory activity, and in particular, HGF and VEGF are important for the inhibition of ROS production.

### (3) Examination of inhibitory effect on endoplasmic reticulum stress using the conditioned medium of the cells

In vitro studies are conducted to verify whether the culture supernatant of MSCs suppressed endoplasmic reticulum stress in the mouse fibroblast cell line NIH3T3 cells, instead of MSCs themselves. A schematic overview of the experiments from the beginning to real-time PCR with 1.5 µg/mL tunicamycin (TM) is shown in Figure 25(A).

In this study, 1 × 10⁵ cells are inoculated into 12-well plates and cultured overnight, and then 500 µL of control medium including suspension of 1.5 µg/ml tunicamycin or 50% hSHED-CM/DMEM (v/v) is added to each well. After overnight incubation at 37 °C, the cells are trypsinized and then collected. Subsequently, RNA is extracted from the cells according to the conventional method. Real-time PCR (herein below sometimes referred to as "q-PCR") is performed using reverse transcriptase. The expression levels of endoplasmic reticulum stress markers (indicator genes for evaluation) are quantified by using mRNA amounts. As the evaluation indicators, the endoplasmic reticulum stress markers, XBP-1, GRP78, and CHOP are used, and as the endogenous control, HPRT is used. The results are shown in Figure 25(B). In Figure 25(B), the vertical axis shows expression levels as the relative ratio, when the control is set to 1.

XBP-1 is known as a negative feedback regulator that blocks transcription of target genes during endoplasmic reticulum stress and has been reported to participate in chaperone induction by binding to the UPR of molecular chaperones such as GRP78 through nuclear transfer during ER stress. GRP78 (HSPA5) is a family member of the HSP70 (heat shock protein 70), which is also called as "immunoglobulin heavy chain-binding protein (BiP)," and it is involved in protein assembly and folding in the endoplasmic reticulum. GRP78 is the endoplasmic reticulum protein that is homeostatically expressed in the endoplasmic reticulum of all eukaryotic cells and has been reported to be associated with the inhibition of cancer cell growth and apoptosis. CHOP is a transcription factor induced under endoplasmic reticulum stress and induces DR5 (death receptor 5) and other proteins in the caspase cascade to activate it to induce apoptosis.

The expression of each endoplasmic reticulum stress marker in NIH3T3 cells cultured in DMEM without supplements was set as 1.0. In cells cultured in DMEM with 1.5 µg/mL of tunicamycin, the expression of each marker was significantly upregulated by approximately 7-fold for XBP-1, 18-fold for GRP78, and 4-fold for CHOP. In contrast, the expression of endoplasmic reticulum stress markers was about 4-fold higher expression of XBP-1, 14-fold higher expression of GRP78, and 3-fold higher expression of CHOP. This indicates significant suppression of the endoplasmic reticulum stress marker expression in cells cultured in the culture medium with hSHED-CM compared to those in the cells without hSHED-CM. The results are shown in Table 19 and Figure 25(B) below.

**Table 19]**

| | Medium used for cell culture | | | Expression level of indicators (relative value) | | |
|---|---|---|---|---|---|---|
| | DMEM | Tunicamycin | hSHED | XBP-1 | GRP78 | CHOP |
| Negative control | + | - | - | 1.0 | 1.0 | 1.0 |
| Positive control | + | + | - | About 7 | About 18 | 4 |
| Sample | + | - | + | About 4* | About 14* | About 3* |

| | | | | | | |
|---|---|---|---|---|---|---|
| *: Significant difference | | | | | | |

### (4) Examination of inhibitory effect on pressure ulcer using the conditioned medium of the cells

As shown in Figure 1, TIMP, a metalloproteinase tissue inhibitor that acts to inhibit collagen and elastin degradation enzymes is rich in hSHED-CM. Since it has been reported that soft tissue acts as a cushion to buffer and disperse pressure against pressure ulcers (see Non-Patent Document 3), an in vivo study was conducted to verify whether administration of hSHED-CM increases the resistance of skin tissue against pressure ulcers.

Similar to the administration schedule 6 in Example 5 (2-6), 100 µL of DMEM or hSHED-CM was administered to the planned sites on which affected area are formed in the control or test groups starting 7 days before I/R treatment (Figure 26(A) and (B)). Dosal skin tissue was collected before I/R treatment to observe the changes of the skin tissue at these sites (Figures 27(A) and (B)). As a result, mice in the test group treated with hSHED-CM tended to have thicker skin tissue in the treated area.

Figure 28(A) shows the time dependent change in the damaged areas, when administered according to the schedule. In the figure, * indicates p<0.05 and ** indicates p<0.01. Figure 28(B) also shows the curing condition of the pressure ulcers of the mice pre-treated with the administration schedule 6. The upper panel shows the target group from Day 2 to Day 16, and the lower panel shows the change in the area of the hSHED-CM-treated group during the same period (Figures 28(A) and (B)).

### (5) Summary

Based on the above, the hSHED-CM seemed to be effective in preventing the aggravation of pressure ulcers. It was also confirmed that hSHED-CM had a certain effect, when administered at any time before or after the formation of pressure ulcers. Furthermore, among the variety of cytokines contained in hSHED-CM, HGF and VEGF seemed to be involved in the prevention and curing of pressure ulcers. In addition, we examined the mechanism of pressure ulcer curing, and had results that hSHED-CM accelerates tissue regeneration through suppression of ROS and alleviation of endoplasmic reticulum stress.

### [Industrial Applicability]

The present invention is useful in the field of the pharmaceuticals.

### Sequence Listing Free Text

SEQ ID NO. 1: Sequence of the DNA fragment containing the gene to be introduced into the cell (1)
SEQ ID NO. 2: Sequence of the DNA fragment containing the gene to be introduced into the cell (2)
SEQ ID NO. 3: Sequence of the DNA fragment containing the gene to be introduced into the cell (3)
SEQ ID NO. 4: Sequence of the DNA fragment containing the gene to be introduced into the cell (4)
SEQ ID NO. 5: Sequence of the DNA fragment containing the gene to be introduced into the cell (5)
SEQ ID NO. 6: Sequence of the DNA fragment containing the gene to be introduced into the cell (6)
SEQ ID NO. 7: Sequence of the DNA fragment containing the gene to be introduced into the cell (7)
SEQ ID NO. 8: Sequence of the DNA fragment containing the gene to be introduced into the cell (8)
SEQ ID NO. 9: Sequence of the DNA fragment containing the gene to be introduced into the cell (9)
SEQ ID NO. 10: Sequence of primer (1) for PCR
SEQ ID NO. 11: Sequence of primer (2) for PCR
SEQ ID NO. 12: Sequence of primer (3) for PCR
SEQ ID NO. 13: Sequence of primer (4) for PCR
SEQ ID NO. 14: Sequence of primer (5) for PCR
SEQ ID NO. 15: Sequence of primer (6) for PCR
SEQ ID NO. 16: Sequence of primer (7) for PCR
SEQ ID NO. 17: Sequence of primer (8) for PCR
SEQ ID NO. 18: Sequence of primer (9) for PCR
SEQ ID NO. 19: Sequence of primer (10) for PCR

[Sequence list]

C:¥Users¥AYAFUNE11_2¥Desktop¥F22CY02WO¥F22CY02WO_ST25.txt

## Claims

1. A preventive and/or therapeutic agent for pressure ulcer comprising conditioned medium of immortalized mesenchymal stem cells as an active ingredient, wherein bmi-1 gene, HPV-E6 gene, HPV-E7 gene, and TERT gene are introduced into the mesenchymal stem cells.

2. The preventive and/or therapeutic agent for pressure ulcer according to claim 1, wherein said TERT is either hTERT or pTERT.

3. The preventive and/or therapeutic agent for pressure ulcer according to claim 1, wherein the mesenchymal stem cells are dental pulp stem cells.

4. The preventive and/or therapeutic agent for pressure ulcer according to claim 1 or claim 3, wherein the dental pulp stem cells are obtained from human or swine.

5. The preventive and/or therapeutic agent for pressure ulcer according to any one of claims 1 to 4, wherein the conditioned medium comprises at least 3 cytokines selected from the group consisting of insulin-like growth factor-binding protein, tissue inhibitor of metalloproteinase, vascular endothelial growth factor, hepatocyte growth factor, tumor necrosis factor receptor II, and osteoprotegerin osteoclast differentiation inhibitory factor in a concentration range from 1 x 10³ to 1 x 10⁶ pg/mL.

6. The preventive and/or therapeutic agent for pressure ulcer according to claim 5, wherein the insulin-like growth factor-binding protein is at least one selected from the group consisting of IGFBP-2, IGFBP-3, IGFBP-4, and IGFBP-6.

7. The preventive and/or therapeutic agent for pressure ulcer according to claim 5, wherein the tissue inhibitor of metalloproteinase is TIMP-1 or TIMP-2.

8. The preventive and/or therapeutic agent for pressure ulcer according to any one of claims 1 to 7, wherein the conditioned medium is any one of forms selected from the group consisting of frozen form, lyophilized powder form, and liquid form.

9. The preventive and/or therapeutic agent for pressure ulcer according to claim 8, wherein the agent further comprises one or more components selected from the group consisting of excipient, pH adjuster, diluent, and buffer.

10. The preventive and/or therapeutic agent for pressure ulcer according to claim 9, wherein dosage form of the preventive and/or therapeutic agent for pressure ulcer is any one selected from the group consisting of injections, injections, ointment, plaster, cream, and transdermal form.

11. A method for restoring pressure ulcer tissue by using the culture composition containing at least three or more cytokines selected from the group consisting of insulin-like growth factor-binding protein, tissue inhibitor of metalloproteinase, vascular endothelial growth factor, hepatocyte growth factor, tumor necrosis factor receptor II and osteoprotegerin osteoclast differentiation inhibitory factor at a concentration range from 1 x 10³ to 1 x 10⁶ pg/mL per each cytokine; wherein the culture composition is acquired by culturing immortalized dental pulp cells induced with a set of the genes of two genes selected from the group consisting of Bmi-1 gene, HPV16-E6 gene and HPV16-E7, and TERT gene.

12. A method for cell regeneration in bedsore tissue by using the culture composition containing at least three or more cytokines selected from the group consisting of insulin-like growth factor-binding protein, tissue inhibitor of metalloproteinase, vascular endothelial growth factor, hepatocyte growth factor, tumor necrosis factor receptor II and osteoprotegerin osteoclast differentiation inhibitory factor at a concentration range from 1 x 10³ to 1 x 10⁵ pg/mL per each cytokine; wherein the culture composition is acquired by culturing immortalized dental pulp cells induced with a set of the genes of two genes selected from the group consisting of Bmi-1 gene, HPV16-E6 gene and HPV16-E7, and TERT gene.
